# EUROPEAN PATENT SPECIFICATION

(11) **EP 1 255 562 B1**
(45) Date of publication and mention of the grant of the patent: **16.01.2008**
(21) Application number: 01921281.0
(22) Date of filing: 13.02.2001
(51) Int. Cl.: A61K 39/39

(54) **USE OF OPRI LIPOPROTEIN FROM PSEUDOMONAS AS A TH1 INDUCING NATURAL ADJUVANT FOR HETEROLOGOUS ANTIGENS**
GEBRAUCH VON OPRI LIPOPROTEIN AUS PSEUDOMONAS ALS TH1 INDUZIERENDES NATÜRLICHES ADJUVANS FÜR HETEROLOGE ANTIGENE
UTILISATION DES LIPOPROTEINES OPRI DE PSEUDOMONAS COMME ADJUVANTS NATURELS INDUISANT UNE REACTION TH1 CONTRE DES ANTIGENES HETEROLOGUES

(30) Priority: 18.02.2000 EP 00200589
(43) Date of publication of application: 13.11.2002
(73) Proprietor: Vlaams Interuniversitair Instituut voor Biotechnologie vzw., 9052 Zwijnaarde (BE)
(72) Inventor: REVETS, Hilde, B-1860 Meise (BE); CORNELIS, Pierre, B-1050 Elsene (BE); DE BAETSELIER, Patrick, B-2600 Berchem (BE)
(86) International application number: PCT/EP2001/001673
(87) International publication number: WO 2001/060404

(56) References cited:
- CORNELIS P ET AL: "DEVELOPMENT OF NEW CLONING VECTORS FOR THE PRODUCTION OF IMMUNOGENIC OUTER MEMBRANE FUSION PROTEINS IN ESCHERICHIA COLI" BIOTECHNOLOGY,GB,BUTTERWORTHS, LONDON, vol. 14, no. 2, 1 February 1996 (1996-02-01), pages 203-208, XP000651289 ISSN: 0740-7378
- COTE-SIERRA J ET AL: "A new membrane-bound OprI lipoprotein expression vector - High production of heterologous fusion proteins in Gram (-) bacteria and the implications for oral vaccination" GENE: AN INTERNATIONAL JOURNAL ON GENES AND GENOMES,GB,ELSEVIER SCIENCE PUBLISHERS, BARKING, vol. 221, no. 1, 9 October 1998 (1998-10-09), pages 25-34, XP004143138 ISSN: 0378-1119 cited in the application
- JONGERT, E. (1) ET AL: "Development of a living bacterial carrier system, based on the lipoprotein I of Pseudomonas aeruginosa, for oral vaccination against food-and-mouth-disease virus." RESEARCH IN IMMUNOLOGY, (JAN., 1998) VOL. 149, NO. 1, PP. 97. MEETING INFO.: EUROCONFERENCE ON NEW TRENDS IN VACCINE RESEARCH AND DEVELOPMENT: ADJUVANTS, DELIVERY SYSTEMS AND ANTIGEN FORMULATIONS PARIS, FRANCE FEBRUARY 26-28, 1998 , XP001013024
- INFANTE-DUARTE, CARMEN ET AL: "Lipopeptides of Borrelia burgdorferi outer surface proteins induce Th1 phenotype development in alpha-beta T-cell receptor transgenic mice." INFECTION AND IMMUNITY, (1997) VOL. 65, NO. 10, PP. 4094-4099., XP000916030
- NABORS G S ET AL: "SWITCH FROM A TYPE 2 TO A TYPE 1 T HELPER CELL RESPONSE AND CURE OF ESTABLISHED LEISHMANIA MAJOR INFECTION IN MICE IS INDUCED BY COMBINED THERAPY WITH INTERLEUKIN 12 AND PENTOSTAM" PROCEEDINGS OF THE NATIONAL ACADEMY OF SCIENCES OF USA,US,NATIONAL ACADEMY OF SCIENCE. WASHINGTON, vol. 92, 1 April 1995 (1995-04-01), pages 3142-3146, XP002060391 ISSN: 0027-8424
- CHU ET AL: "CpG Oligodeoxynucleotides act as adjuvants that switch on T helper 1 (Th1) immunity" J. EXP. MED, vol. 186, 1997, pages 1623-1631,
- MCCLUSKIE ET AL: "Route and Method of Delivery of DNA Vaccine influence immune responses in mice and non-human primates" MOLECULAR MEDICINE, vol. 5, 1999, pages 287-300,
- REVETS ET AL: "Lipoprotein I, a TLR2/4 ligand modulates Th2-driven allergic immune responses" THE JOURNAL OF IMMUNOLOGY, vol. 174, 2005, pages 1097-1103,

### SEQUENCE LISTING

<110> Vlaams Interuniversitair Instituut voor Biotechnol
<120> Th1 inducing natural adjuvant for heterologous antigens
<130> HRE/ADJ/V051
<140>
   <141>
<150> EP00200589.0
   <151> 2000-02-18
<160> 6
<170> PatentIn Ver. 2.1
<210> 1
   <211> 57
   <212> PRT
   <213> Pseudomonas aeruginosa
<400> 1
   Cys Ser Ser His Ser Lys Glu Thr Glu Ala Arg Leu Thr Ala Thr Glu
     1               5                  10                  15
   Asp Ala Ala Ala Arg Ala Gln Ala Arg Ala Asp Glu Ala Tyr Arg Lys
                20                  25                  30
   Ala Asp Glu Ala Leu Gly Ala Ala Gln Lys Ala Gln Gln Thr Ala Asp
            35                  40                  45
   Glu Ala Asn Glu Arg Ala Leu Arg Met
        50                  55
<210> 2
   <211> 1884
   <212> DNA
   <213> Artificial Sequence
<220>
   <223> Description of Artificial Sequence: OprI-3D-FMDV15 fusion
<220>
   <221> CDS
   <222> (1)..(1884)
<400> 2
   atg aac aac gtt ctg aaa ttc tct gct ctg gct ctg gct gct gtt ctg 48
   Met Asn Asn Val Leu Lys Phe Ser Ala Leu Ala Leu Ala Ala Val Leu
   1 5 10 15
   gcc acc ggt tgc agc agc cac tcc aaa gaa acc gaa gct cgt ctg acc 96
   Ala Thr Gly Cys Ser Ser His Ser Lys Glu Thr Glu Ala Arg Leu Thr
   20 25 30
   gct acc gaa gac gca gct gct cgt gct cag gct cgc gct gac gaa gcc 144
   Ala Thr Glu Asp Ala Ala Ala Arg Ala Gln Ala Arg Ala Asp Glu Ala
   35 40 45
   tat cgc aag gct gac gaa gct ctg ggc gct gct cag aaa gct cag cag 192
   Tyr Arg Lys Ala Asp Glu Ala Leu Gly Ala Ala Gln Lys Ala Gln Gln
   50 55 60
   acc gct gac gag gct aac gag cgt gcc ctg cgc atg cag atc atc ggg 240
   Thr Ala Asp Glu Ala Asn Glu Arg Ala Leu Arg Met Gln Ile Ile Gly
   65 70 75 80
   ttg att gtg gac acc aga gat gtg gaa gag cgc gtt cac gtg atg cgc 288
   Leu Ile Val Asp Thr Arg Asp Val Glu Glu Arg Val His Val Met Arg
   85 90 95
   aaa acc aag ctt gca ccc acc gtt gca cac ggt gtg ttc aac ccc gag 336
   Lys Thr Lys Leu Ala Pro Thr Val Ala His Gly Val Phe Asn Pro Glu
   100 105 110
   ttt ggg ccc gct gcc ttg tcc aac aag gac ccg cgt ctg aac gag ggt 384
   Phe Gly Pro Ala Ala Leu Ser Asn Lys Asp Pro Arg Leu Asn Glu Gly
   115 120 125
   gtt gtc ctc gac gaa gtc atc ttc tcc aaa cac aag gga gac aca aag 432
   Val Val Leu Asp Glu Val Ile Phe Ser Lys His Lys Gly Asp Thr Lys
   130 135 140
   atg tct gag gag gac aaa gcg ctg ttc cgc cgc tgc gct gct gac tac 480
   Met Ser Glu Glu Asp Lys Ala Leu Phe Arg Arg Cys Ala Ala Asp Tyr
   145 150 155 160
   gcg tca cgc ttg cac agc gtg ttg ggc aca gca aat gcc cca ctg agc 528
   Ala Ser Arg Leu His Ser Val Leu Gly Thr Ala Asn Ala Pro Leu Ser
   165 170 175
   atc tac gag gca atc aag ggt gtc gac gga ctc gac gcc atg gaa cca 576
   Ile Tyr Glu Ala Ile Lys Gly Val Asp Gly Leu Asp Ala Met Glu Pro
               180                 185                 190
   gac act gcg ccc ggc ctc ccc tgg gcc ctc cag ggt aaa cgc cgc ggc    624
   Asp Thr Ala Pro Gly Leu Pro Trp Ala Leu Gln Gly Lys Arg Arg Gly
           195                 200                 205
   gcg ctc atc gac ttc gag aac ggc acg gtc gga ccc gaa gtt gag gct    672
   Ala Leu Ile Asp Phe Glu Asn Gly Thr Val Gly Pro Glu Val Glu Ala
       210                 215                 220
   gcc ctg aag ctc atg gag aag aga gaa tac aaa ttt gtt tgt cag acc    720
   Ala Leu Lys Leu Met Glu Lys Arg Glu Tyr Lys Phe Val Cys Gln Thr
   225                 230                 235                 240
   ttc ctg aag gac gag att cgc ccg ttg gag aaa gta cgt gcc ggt aag    768
   Phe Leu Lys Asp Glu Ile Arg Pro Leu Glu Lys Val Arg Ala Gly Lys
                   245                 250                 255
   act cgc att gtc gac gtc ctg ccc gtt gag cac att ctt tac acc agg    816
   Thr Arg Ile Val Asp Val Leu Pro Val Glu His Ile Leu Tyr Thr Arg
               260                 265                 270
   atg atg att ggc aga ttt tgt gca cag atg cac tca aat aac gga ccg    864
   Met Met Ile Gly Arg Phe Cys Ala Gln Met His Ser Asn Asn Gly Pro
           275                 280                 285
   caa att ggc tca gcg gtc ggt tgc aac cct gat gtt gat tgg cag aga    912
   Gln Ile Gly Ser Ala Val Gly Cys Asn Pro Asp Val Asp Trp Gln Arg
       290                 295                 300
   ttt ggc aca cac ttc gcc cag tac aga aac gtg tgg gat gtg gac tat    960
   Phe Gly Thr His Phe Ala Gln Tyr Arg Asn Val Trp Asp Val Asp Tyr
   305                 310                 315                 320
   tcg gcc ttt gat gct aat cac tgt agt gat gcc atg aac atc atg ttt    1008
   Ser Ala Phe Asp Ala Asn His Cys Ser Asp Ala Met Asn Ile Met Phe
                   325                 330                 335
   gag gag gtg ttt cgc acg gag ttc ggc ttc cac ccg aat gct gag tgg    1056
   Glu Glu Val Phe Arg Thr Glu Phe Gly Phe His Pro Asn Ala Glu Trp
               340                 345                 350
   atc ctg aag act ctt gtg aac acg gaa cac gcc tat gag aac aaa cgc    1104
   Ile Leu Lys Thr Leu Val Asn Thr Glu His Ala Tyr Glu Asn Lys Arg
           355                 360                 365
   atc act gtt gga ggc gga atg ccg tct ggt tgc tcc gca aca agc atc    1152
   Ile Thr Val Gly Gly Gly Met Pro Ser Gly Cys Ser Ala Thr Ser Ile
       370                 375                 380
   atc aac aca att ttg aac aac atc tac gtg ctc tac gcc ctg cgt aga     1200
   Ile Asn Thr Ile Leu Asn Asn Ile Tyr Val Leu Tyr Ala Leu Arg Arg
   385                 390                 395                 400
   cac tat gag gga gtt gag ctg gac aca tac acc atg atc tcc tac gga     1248
   His Tyr Glu Gly Val Glu Leu Asp Thr Tyr Thr Met Ile Ser Tyr Gly
                   405                 410                 415
   gac gac atc gtg gtg gca agt gat tat gat ttg gac ttc gag gct ctc     1296
   Asp Asp Ile Val Val Ala Ser Asp Tyr Asp Leu Asp Phe Glu Ala Leu
               420                 425                 430
   aag ccc cac ttt aaa tcc ctt ggc caa acc atc act cca gct gac aaa     1344
   Lys Pro His Phe Lys Ser Leu Gly Gln Thr Ile Thr Pro Ala Asp Lys
           435                 440                 445
   agc gac aaa ggt ttt gtt ctt ggt cac tcc att acc gat gtc act ttc     1392
   Ser Asp Lys Gly Phe Val Leu Gly His Ser Ile Thr Asp Val Thr Phe
       450                 455                 460
   ctc aaa agg cac ttc cac atg gac tat gga act ggg ttt tac aaa cct     1440
   Leu Lys Arg His Phe His Met Asp Tyr Gly Thr Gly Phe Tyr Lys Pro
   465                 470                 475                 480
   gtg atg gcc tca aag acc ctt gag gct atc ctc tcc ttt gca cgc cgt     1488
   Val Met Ala Ser Lys Thr Leu Glu Ala Ile Leu Ser Phe Ala Arg Arg
                   485                 490                 495
   ggg acc ata cag gag aag ttg atc tcc gtg gca gga ctc gcc gtc cac     1536
   Gly Thr Ile Gln Glu Lys Leu Ile Ser Val Ala Gly Leu Ala Val His
               500                 505                 510
   tct gga cca gac gag tac cgg cgt ctc ttt gag cct ttc caa ggt ctc     1584
   Ser Gly Pro Asp Glu Tyr Arg Arg Leu Phe Glu Pro Phe Gln Gly Leu
           515                 520                 525
   ttt gag att cca agc tac aga tca ctt tac ctg cgt tgg gtg aac gcc     1632
   Phe Glu Ile Pro Ser Tyr Arg Ser Leu Tyr Leu Arg Trp Val Asn Ala
       530                 535                 540
   gtg tgc ggt gac gcg ctg gtt ccg cgt gga tcc tgc cgg cac aaa caa     1680
   Val Cys Gly Asp Ala Leu Val Pro Arg Gly Ser Cys Arg His Lys Gln
   545                 550                 555                 560
   aaa ata gtg gcg cca gta aaa caa aca cta cca cca tca aac cta cga     1728
   Lys Ile Val Ala Pro Val Lys Gln Thr Leu Pro Pro Ser Asn Leu Arg
                   565                 570                 575
   gga gat cta caa gta cta gca caa aaa gtg gcg cgc aca cta cca tgc    1776
   Gly Asp Leu Gln Val Leu Ala Gln Lys Val Ala Arg Thr Leu Pro Cys
               580                 585                 590
   ggg aat tct gtt aac cgc atg ctg gaa aaa gcc agc cgc aag cat ggc    1824
   Gly Asn Ser Val Asn Arg Met Leu Glu Lys Ala Ser Arg Lys His Gly
           595                 600                 605
   tgc agc caa gct tgg ctg ttt tgg cgg atg aga gaa gat ttt cag ctt    1872
   Cys Ser Gln Ala Trp Leu Phe Trp Arg Met Arg Glu Asp Phe Gln Leu
       610                 615                 620
   gat aca gat taa                                                    1884
   Asp Thr Asp
   625
<210> 3
   <211> 627
   <212> PRT
   <213> Artificial Sequence
   <223> Description of Artificial Sequence: OprI-3D-FMDV15 fusion
<400> 3
   Met Asn Asn Val Leu Lys Phe Ser Ala Leu Ala Leu Ala Ala Val Leu
    1               5                  10                  15
   Ala Thr Gly Cys Ser Ser His Ser Lys Glu Thr Glu Ala Arg Leu Thr
               20                  25                  30
   Ala Thr Glu Asp Ala Ala Ala Arg Ala Gln Ala Arg Ala Asp Glu Ala
           35                  40                  45
   Tyr Arg Lys Ala Asp Glu Ala Leu Gly Ala Ala Gln Lys Ala Gln Gln
       50                  55                  60
   Thr Ala Asp Glu Ala Asn Glu Arg Ala Leu Arg Met Gln Ile Ile Gly
   65                  70                  75                  80
   Leu Ile Val Asp Thr Arg Asp Val Glu Glu Arg Val His Val Met Arg
                   85                  90                  95
   Lys Thr Lys Leu Ala Pro Thr Val Ala His Gly Val Phe Asn Pro Glu
               100                 105                 110
   Phe Gly Pro Ala Ala Leu Ser Asn Lys Asp Pro Arg Leu Asn Glu Gly
           115                 120                 125
   Val Val Leu Asp Glu Val Ile Phe Ser Lys His Lys Gly Asp Thr Lys
       130                 135                 140
   Met Ser Glu Glu Asp Lys Ala Leu Phe Arg Arg Cys Ala Ala Asp Tyr
   145                 150                 155                 160
   Ala Ser Arg Leu His Ser Val Leu Gly Thr Ala Asn Ala Pro Leu Ser
                   165                 170                 175
   Ile Tyr Glu Ala Ile Lys Gly Val Asp Gly Leu Asp Ala Met Glu Pro
               180                 185                 190
   Asp Thr Ala Pro Gly Leu Pro Trp Ala Leu Gln Gly Lys Arg Arg Gly
           195                 200                 205
   Ala Leu Ile Asp Phe Glu Asn Gly Thr Val Gly Pro Glu Val Glu Ala
       210                 215                 220
   Ala Leu Lys Leu Met Glu Lys Arg Glu Tyr Lys Phe Val Cys Gln Thr
   225                 230                 235                 240
   Phe Leu Lys Asp Glu Ile Arg Pro Leu Glu Lys Val Arg Ala Gly Lys
                   245                 250                 255
   Thr Arg Ile Val Asp Val Leu Pro Val Glu His Ile Leu Tyr Thr Arg
               260                 265                 270
   Met Met Ile Gly Arg Phe Cys Ala Gln Met His Ser Asn Asn Gly Pro
           275                 280                 285
   Gln Ile Gly Ser Ala Val Gly Cys Asn Pro Asp Val Asp Trp Gln Arg
       290                 295                 300
   Phe Gly Thr His Phe Ala Gln Tyr Arg Asn Val Trp Asp Val Asp Tyr
   305                 310                 315                 320
   Ser Ala Phe Asp Ala Asn His Cys Ser Asp Ala Met Asn Ile Met Phe
                   325                 330                 335
   Glu Glu Val Phe Arg Thr Glu Phe Gly Phe His Pro Asn Ala Glu Trp
               340                 345                 350
   Ile Leu Lys Thr Leu Val Asn Thr Glu His Ala Tyr Glu Asn Lys Arg
           355                 360                 365
   Ile Thr Val Gly Gly Gly Met Pro Ser Gly Cys Ser Ala Thr Ser Ile
       370                 375                 380
   Ile Asn Thr Ile Leu Asn Asn Ile Tyr Val Leu Tyr Ala Leu Arg Arg
   385                 390                 395                 400
   His Tyr Glu Gly Val Glu Leu Asp Thr Tyr Thr Met Ile Ser Tyr Gly
                   405                 410                 415
   Asp Asp Ile Val Val Ala Ser Asp Tyr Asp Leu Asp Phe Glu Ala Leu
               420                 425                 430
   Lys Pro His Phe Lys Ser Leu Gly Gln Thr Ile Thr Pro Ala Asp Lys
           435                 440                 445
   Ser Asp Lys Gly Phe Val Leu Gly His Ser Ile Thr Asp Val Thr Phe
       450                 455                 460
   Leu Lys Arg His Phe His Met Asp Tyr Gly Thr Gly Phe Tyr Lys Pro
   465                 470                 475                 480
   Val Met Ala Ser Lys Thr Leu Glu Ala Ile Leu Ser Phe Ala Arg Arg
                   485                 490                 495
   Gly Thr Ile Gln Glu Lys Leu Ile Ser Val Ala Gly Leu Ala Val His
               500                 505                 510
   Ser Gly Pro Asp Glu Tyr Arg Arg Leu Phe Glu Pro Phe Gln Gly Leu
           515                 520                 525
   Phe Glu Ile Pro Ser Tyr Arg Ser Leu Tyr Leu Arg Trp Val Asn Ala
       530                 535                 540
   Val Cys Gly Asp Ala Leu Val Pro Arg Gly Ser Cys Arg His Lys Gln
   545 550 555 560
   Lys Ile Val Ala Pro Val Lys Gln Thr Leu Pro Pro Ser Asn Leu Arg
   565 570 575
   Gly Asp Leu Gln Val Leu Ala Gln Lys Val Ala Arg Thr Leu Pro Cys
   580 585 590
   Gly Asn Ser Val Asn Arg Met Leu Glu Lys Ala Ser Arg Lys His Gly
   595 600 605
   Cys Ser Gln Ala Trp Leu Phe Trp Arg Met Arg Glu Asp Phe Gln Leu
   610 615 620
   Asp Thr Asp
   625
<210> 4
   <211> 35
   <212> DNA
   <213> Artificial Sequence
<220>
   <223> Description of Artificial Sequence: primer
<400> 4
   gcgcggatcc tgcagcagcc actccaaaga aaccg 35
<210> 5
   <211> 32
   <212> DNA
   <213> Artificial Sequence
<220>
   <223> Description of Artificial Sequence: primer
<100> 5
   ctttttcggt cggcgttcat tattcgaacg cg 32
<210> 6
   <211> 8
   <212> PRT
   <213> Artificial Sequence
<220>
   <223> Description of Artificial Sequence: OVA-derived minimal CTL peptide
<400> 6
   Ser Ile Ile Asn Phe Glu Lys Leu
   1 5

## Description

The present application relates to the use of the major Oprl lipoprotein of *Pseudomonas aeruginosa* to elicit a Type-1 immune response towards a heterologous antigen. The invention as defined in the claims relates specifically to the use of Oprl - antigen fusion proteins to elicit said Type-1 response. More particularly, pharmaceutical formulations comprising Oprl and/or Oprl fusion proteins, optionally together with a suitable excipient, to stimulate the Th1 dependent, cellular immune response are disclosed. Upon T-Cell Receptor (TCR) - ligation, Th0 cells differentiate into distinct subsets characterized by their functions and cytokine production profiles (Mosmann and Coffman, 1989). Th1 lymphocytes, characterized by the production of IL-2, IFN-γ and TNF-β, contribute to cellular immunity whereas Th2 lymphocytes, producing IL-4, IL-5 and IL-10 are mainly involved in humoral immunity. The generation of cell-mediated immunity against many infectious pathogens relies on the induction of the innate immune system, which in turn generates appropriate signals for adaptive immune responses (Fearon and Locksley, 1996). Bacterial lipoproteins are among others, molecules that stimulate cells of the innate immune system to produce cytokines such as TNF-α (Radolf *et al.,* 1991; Vidal *et al.,* 1998) and IL-12 (Brightbill *et al.,* 1999). Hereby, bacterial lipoproteins activate innate immune cells via Toll-like receptors (Brightbill *et al.,* 1999; Aliprantis *et al.,* 1999) and their signaling activity resides in the NH2-terminal triacylated lipopeptide region (Erdile *et al.,* 1993; Weis *et al.,* 1994). The potent capacity of bacterial lipoproteins and/or lipopeptides to induce the production of IL-12 (Brightbill *et al.,* 1999), a key signal of the innate immune system, may in turn trigger the development of adaptive immune responses.

Numerous examples of the consequences on disease outcome of skewed Th1 to Th2 ratios have been reported. Polarized Th2 responses have been implicated in pathological situations, such as *Leishmania major* infection (Heinzel *et al.,* 1991; Nabors *et al.,* 1995), TBC (de Jong *et al.,* 1997), human leprosy (Yamamura *et al.,* 1991) and mycotic infections (Murphy *et al.,* 1994). The contribution of Th1 cells relative to Th2 cells to the developing autoimmune response determines for a larger part whether or not this response leads to clinical disease (Racke *et al.,* 1994; Racke *et al.,* 1995; Leonard *et al.,* 1995). In allergic asthma, a predominant Th2 type response has been noted (Vogel, 1997). Also the chronic autoimmune graft-versus-host disease, which develops after the administration of mismatched lymphoid cells, can be prevented by switching a Th2 response to a Th1 response through administration of IFN-γ at the time of cellular transfer (Donckier *et al.,* 1994).

Several methods have been proposed to modulate the Th1/Th2 response. W09726883 describes the use of ribavirin3 to treat imbalances in lymphokine expression. WO9848805 discloses chemical compounds that suppress the Th2 type response and can be used for treating or preventing a disease caused by abnormal activation of Th2 type immune response, such as asthma, allergic dermatitis, allergic rhinitis or systemic lupus erythematosus. However, those chemical compounds may have unwanted side effects. WO9921968 describes the use of macrophages in the function of antigen presenting cells to redirect the balance of Th1/Th2 cell subsets during an immune response. Although the latter method is more specific, it is complicated because personalized immortalized macrophage clones should be made for each patient to be treated.

It has been demonstrated that bacterial lipoproteins may also be useful in modulating the Th1/Th2 immune response. The synthetic lipid moiety analogue of bacterial lipoproteins (i.e. the tripalmitoyl-s-glyceryl-cysteine or Pam3Cys) was reported to increase the immunogenicity of heterologous antigens (Bessler *et al.,* 1985; Lex *et al.,* 1986; Deres *et al.,* 1989; BenMohamed *et al.,* 1997). Lipopeptides derived from the outer surface lipoproteins of *Borrelia burgdorferi* were reported to induce Th1 phenotype development (Infante-Duarte and Kamradt, 1997). It has been reported that fusion proteins between the major Oprl lipoprotein of *Pseudomonas aeruginosa* and heterologous peptides or proteins were found to be highly immunogenic as evidenced by the induction of strong humoral and cytotoxic T cell responses without the need of adjuvants (Cornelis *et al.,* 1996; Leitao *et al.,* 1998). Although it is clear from these data that Oprl may be useful as an adjuvant, there is no indication that Oprl can modulate the immune response. Moreover, Ino *et al.* (1999) describes that Oprl can act as a strong inducer of cytokines in mouse bone marrow cells. When purified Oprl was added to mouse bone marrow cells, an induction of TNFα, IL-1a, IL-1b, IL-6 and granulocyte-macrophage colony stimulating was seen. However, IL-2, IFN-γ and TNF-β, typical for a Th1 response, were not detected.

Surprisingly it is demonstrated in this invention that the Oprl-antigen fusion elicits a Type-1 immune response towards the heterologous antigen that is fused to Oprl, even in the case where the antigen on its own does not induce a Th1 type response, or induces the Th1 response only to a limited extent. It is especially unexpected that this response is not only directed towards Oprl itself, but also to the heterologous antigen, as is demonstrated by analysis of the antibody titers. The induction of the Type-1 immune response can be clearly allocated to the lipid tail of Oprl.

Therefore, one aspect of this invention is the use of Oprl, or functional fragments thereof, as an adjuvant to obtain a Th1 type immune response against a heterologous antigen as defined in claim 1. A preferred embodiment of the invention is said use, whereby Oprl or a functional fragment thereof is fused to the heterologous antigen.

WO 9504079 describes the use of Oprl to expose proteins on the surface of host cells. In the present invention, it is unexpectedly demonstrated that host cells, presenting a heterologous antigen fused to Oprl, can stimulate the Th1 response towards the heterologous antigen in a similar way as if the purified Oprl-antigen fusion protein is used.

Therefore, another aspect of the invention is the use of a host cell, expressing an Oprl-heterologous antigen fusion protein to obtain a Th1 type response against said heterologous antigen defined in claim 1.

Another aspect of the invention is the use of Oprl and /or the use of an Oprl-heterologous antigen fusion protein and/or the use of a host cell, expressing an Oprl-heterologous antigen fusion protein to treat a disease in which the natural Th1 response is insufficient, and/or the response is polarized towards a Th2 response. These diseases are: TBC, leprosy, mycotic infections, allergic asthma, and several autoimmune diseases such as chronic autoimmune graft-versus-host disease and infection with a rotavirus.

Still another aspect of the invention is a process for the manufacture of a pharmaceutical composition characterized in the use of Oprl and/or Oprl fused to a heterologous antigen and/or a host cell expressing an Oprl-heterologous antigen fusion, according to the invention.

Still another aspect of the invention is a pharmaceutical composition to treat diseases, as defined in claim 1, which the natural Th1 response is insufficient, comprising Oprl and/or Oprl fused to a heterologous antigen and/or a host cell expressing an Oprl-heterologous antigen fusion protein, optionally together with a suitable excipient.

### Definitions

The following definitions are set forth to illustrate and define the meaning and scope of the various terms used to describe the invention herein:
*Functional fragment* of Oprl means any fragment that has still the adjuvant capacity and Th1 inducing ability. Preferentially, the functional fragment is comprising at the least the 4 amino terminal amino acids of the sequence shown in SEQ.lD.N°1, including the lipid modification, more preferentially at the least the 10 amino terminal amino acids of the sequence shown in SEQ.ID.N°1, including the lipid modification and most preferentially the functional fragments is comprising the 57 amino terminal amino acids of the mature Oprl protein, as shown in SEQ.ID.N°1, including the lipid modification.
*Functional fragment* of an antigen means a part of said antigen that still has antigenic activity and is containing at least one epitope.
*Heterologous antigen* means an antigen that has at least one epitope that differs from the epitopes of Oprl.
*Host cell* means any host cell in which the Oprl-heterologous antigen fusion protein can be expressed and whereby the antigen is presented on the surface of said host cell. Preferentially, the host cell is a bacterium, more preferentially, the host cell is a gram negative bacterium, even more preferentially the host cell is *Escherichia coli, Alcaligenes eutrophus* or *Salmonella typhimurium.*

### Brief description of the figures

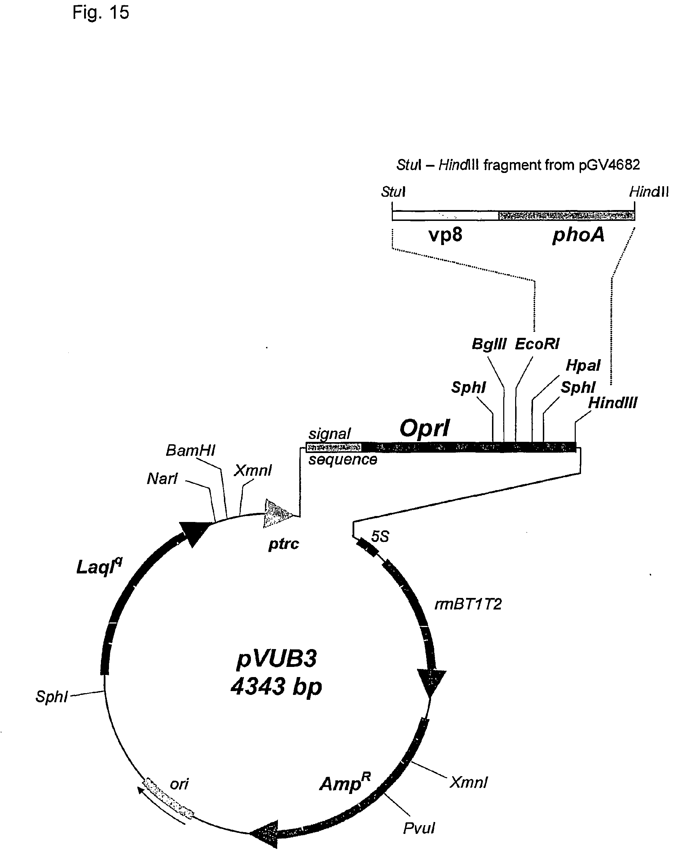

Figure 1: Plasmid map of pVUB3.
Figure 2: Plasmid map of pCIMM2
Figure 3: Formulations of the three recombinant Gp63 preparations used in this study. L-OprlCOOHgp63: lipidated Oprl/COOHgp63 fusion protein NL-OprlCOOHgp63: non-lipidated Oprl/COOHgp63 fusion protein COOHgp63: 6xhis-tagged COOHgp63
Figure 4: Release of IFN-γ and IL-10 from lymph nodes of L-OprlCOOHgp63, NL-OprlCOOHgp63 and COOHgp63 immunized-mice. Production of IFN-γ and IL-10 was quantified in the lymph nodes of BALB/c (**a**) and C57BU6 (**b**) mice 7 days after immunization
   Results show values of pooled sacral lymph nodes from five mice, representative of two similar experiments.
Figure 5: Anti-Gp63 antibody responses in mice immunized with L-OprICOOHgp63, NL-OprlCOOHgp63 or COOHgp63. IgG antibody titers against COOHgp63 in sera from BALB/c (a) and C57BU6 (b) mice 10 days after mice received the third injection of the mentioned protein. Results of end-point ELISA titers are from pooled sera of five mice. The experiment was repeated twice and similar results were obtained.
Figure 6: The lipid tail of L-OprlCOOHgp63 is required to induce TNF-α release by peritoneal macrophages either activated or not with 100 units/ml IFN-γ. The data are representative for two independent experiments.
Figure 7: The lipoprotein-induced Type-1 immune response is affected in TNF-α knockout mice (TNF-α^{-/-}). IFN-γ and IL-10 production in sacral lymph node (**a, c**) and spleen (**b, d**) cells from mice immunized with one (**a, b**) or three doses (**c, d**) of L-OprlCOOHgp63. IgG antibody titers against COOHgp63 in sera from BALB/c, C57BU6 and C57BU6 TNF-α^{-/-} mice, 10 days after mice received the third dose of L-OpriCOOHgp63 (e). Results show end-point ELISA titers from pooled sera samples of five mice. Similar results were obtained in a second independent experiment.
Figure 8: The Oprl-based COOHgp63 lipoprotein protects BALB/c mice against *Leishmania* challenge. Groups of 15 mice were vaccinated subcutaneously three times with the lipidated L-OprlCOOHgp63, the non-lipidated NL-OprlCOOHgp63 or COOHgp63. Controls were injected with buffer. Mice were infected with 10⁶ live promastigotes 10 days after the last immunization and lesion development was monitored weekly.
Figure 9: Plasmid map of pVUB3:3D15.
Figure 10: IFN-γ(A) and IL-10 (B) production in spleen cells from mice immunized once with SL3261(pVUB3:3D15). Splenic lymphocytes were restimulated with SL3261 lysate (SL3261), ovalbumin (OVA), non-lipidated Oprl (NL-Oprl) or 6x his-3D protein (3D).
Figure 11: Preimmune and immune humoral isotype responses in mice immunized once with SL3261 (pVUB3:3D15). The abbreviations are the same as in Figure 9.
Figure 12: (A) Antibody response measured in serum and (B) Production of IFN-γ in spleen cells from BALB/c mice, immunised 3 times at a 10 days interval, 2 and 12 weeks after immunisation with L-OprlCOOHgp63 (indicated as Oprl-Cgp63).
Figure 13: (A) Antibody response measured in serum and (B) Production of IFN-γ in spleen cells from BALB/c mice, immunised 3 times at a 10 days interval, 2 and 12 weeks after immunisation with either the L-OprlCOOHgp63 fusion (indicated as Oprl-Cgp63), or a mixture of L-Oprl and COOHgp63 (indicated as Oprl + Cgp63).
Figure 14: Lysis of OVA₂₅₇₋₂₆₄ peptide-loaded RMA-S cells by cytotoxic T cell lymphocytes, induced against the OVA₂₅₇₋₂₆₄ (SIINFEKL) epitope, in the presence of various adjuvants.
   Oprl + CTL: 1 µg Oprl + 5 µg OVA ₂₅₇₋₂₆₄ MHC class I (K^{b}-restricted) peptide
   Oprl + CTL + Th: 1 µg Oprl + 5 µg OVA 257-264 MHC class! (K^{b}-restricted) peptide + 5 µg OVA₂₆₅₋₂₈₀ (MHC class 11 (I-Ab-restricted) Tₕ peptide
   PBS + CTL: PBS + 5 µg OVA 257-264 MHC class I (K^{b}-restricted) peptide
   CFA + CTL: CFA + 5 µg OVA 257-264 MHC class I (K^{b}-restricted) peptide
   Oprl + OVA: 1 µg L-Oprl + 1 µg OVA protein
   PBS + OVA: 1 µg OVA in PBS
   RMA-S + OVA: RMA-S cells loaded with OVA₂₅₇₋₂₆₄ peptide
   RMA-S: non-loaded RMA-S cells
Figure 15: plasmid map of pVUB3:VP8

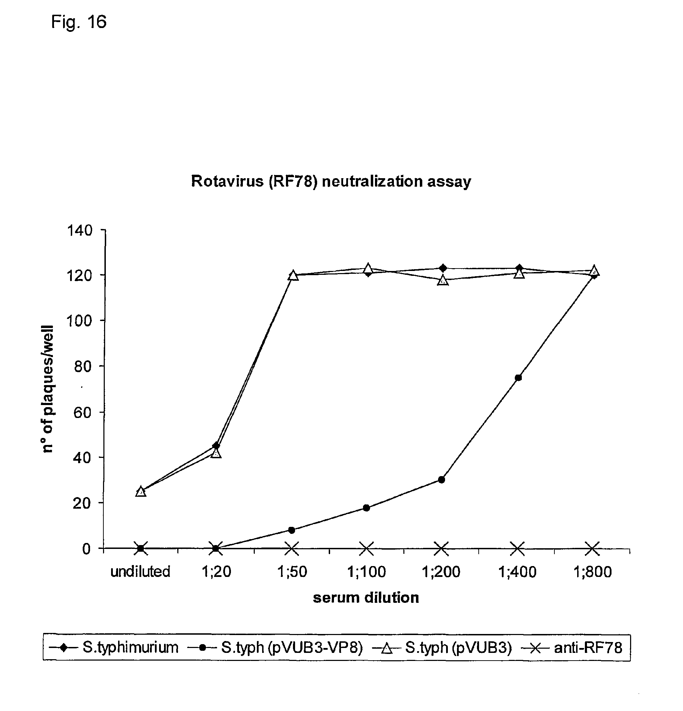

Figure 16: Rotavirus strain RF78 neutralisation assay using sera collected from mice immunized with *Salmonella typhimurium* χ4046 (S.typhimurium), *S. typhimurium* χ4046 transformed with pVUB3 (S.typh (pVUB3)) and *S. typhimurium* χ4046 transformed with pVUB3-VP8 (S.typh (pVUB3-VP8)). Anti-RF78 represents the positive control using polyclonal anti-RF78 antibodies (obtained from Dr. Cohen, INRA, France).

### Examples

### Materials and methods to the examples

### Mice.

Female BALB/c, C57BU6 and LPS-resistant C3H/HeJ mice of 6-8 weeks of age were obtained from Harlan Nederland (Horst, The Netherlands). C57BU6 TNF-α knockout (TNF-α^{-/-}) mice were obtained from the National Institute of Animal Health, Tsukuba City, Japan (Taniguchi *et al.,* 1997) and maintained in our animal facility.

### Construction of pVUB3.

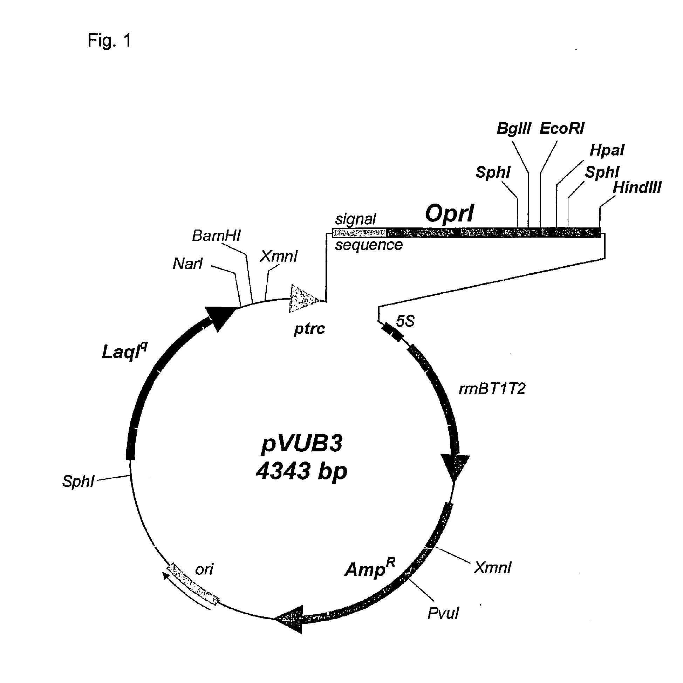

The construction of the pVUB3 expression plasmid has been described in detail by Cote-Sierra et al. (1998). A plasmid map is depicted in Figure 1.

### Construction of the expression vector pCIMM2.

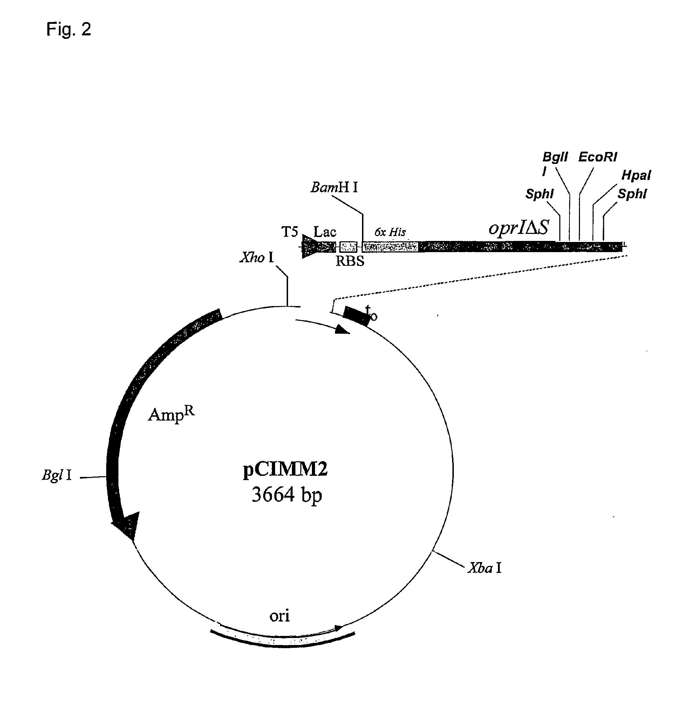

The *P. aeruginosa* mature *oprl* gene sequence contained in plasmid pVUB3 (Cote-Sierra *et al.,* 1998) was amplified by PCR with the following primers 5'-GCGCGGATCCTGCAGCAGCCACTCCAAAGAAACCG-3' and 3'-CTTTTTCGGTCGGCGTTCATTATTCGAACGCG-5'. Amplified DNA was purified, digested with *BamHI* and *HindIII,* and cloned downstream of a sequence encoding an oligo-histidine peptide of six residues in the expression vector pQE-8 (Qiagen GmbH, Germany), devoided of its *EcoRI* site. The resulting construct, pCIMM2, was transformed into JM109 competent cells. In pCIMM2, the *oprI* gene is devoided of its signal sequence and consequently, cannot be transported to the bacterial outer membrane. As such, the protein will remain in the cytosol as a non-lipidated protein (NL-Oprl). Due to the 6xhis tail at its 5' end, the protein can be purified by Immobilized Metal Affinity Chromatography (IMAC). In addition, the expression plasmid can be used for further subcloning of heterologous, antigens into the NL-Oprl sequence in order to create non-lipidated Oprl/heterologous antigen fusion proteins. A plasmid map of pCIMM2 is depicted in Figure 2.

### Generation of lipidated (L-Oprl) recombinant antigens.

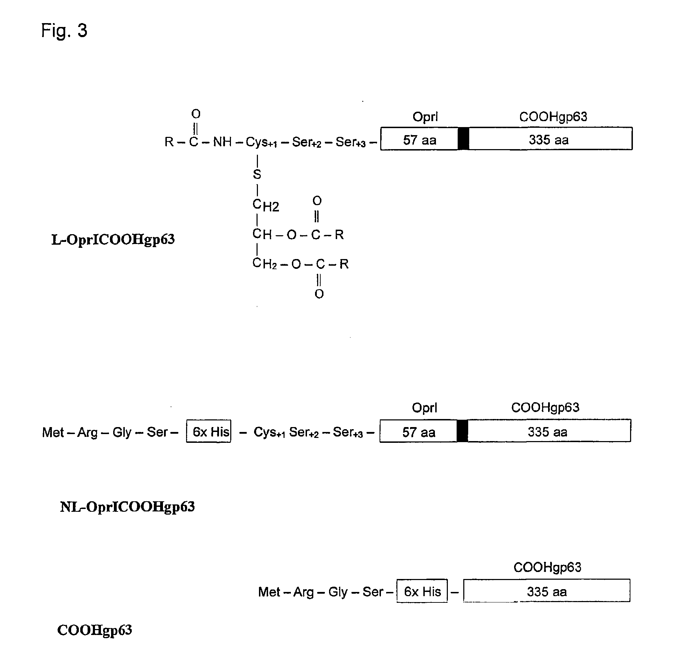

The generation of the lipidated L-OprlCOOHgp63 fusion construct was described in detail previously (Cote-Sierra *et al.,* 1998) (Fig.3). The ligation mixture was subsequently transformed into a chemocompetent *E. coli* host using standard procedures.

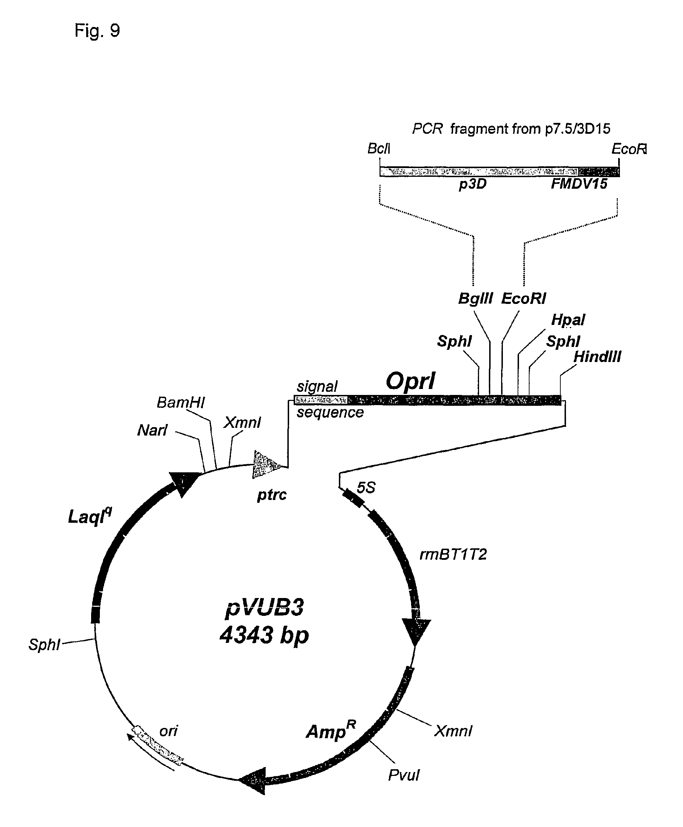

A lipidated L-Oprl/3D-FMDV15 fusion antigen was constructed as follows: the plasmid P7.5/3D15 containing the chimaeric 3D-FMDV15 gene (a complex B-cell/T-cell construct consisting of the FMDV-15 peptide colineary linked with the T-cell immunodominant non-structural protein 3D) was kindly provided by Dr. M. Parkhouse (Institute for Animal Health, Pirbright, U.K.). The chimaeric gene was amplified as a *BcII-EcoRl* PCR fragment and directionally cloned into the pVUB3 expression vector restricted with *Bglll-EcoRl* (see Fig. 9; SEQ. ID. N°2). The ligation mixture was subsequently transformed into a chemocompetent *E. coli* host using standard procedures.

The VP8 gene was amplified by PCR from the murine rotavirus strain EW (G3P17) and cloned into plasmid pGV4684 as fusion with phoA. Subsequently, the VP8-phoA fragment was ligated as a *StuI-HindIII* fragment into pVUB3, digested with EcoR/(fill-in)-*HindIII* (Fig. 15). The ligation mixture was subsequently transformed into a chemocompetent *E. coli* host using standard procedures.

### Generation of non-lipidated (NL-Oprl) recombinant antigens.

The recombinant vector producing the 6xhis-non-lipidated NL-OprlCOOHgp63 protein (Fig.3) was constructed by introducing the *BgIII-HindIII* COOHgp63 DNA fragment (generated by digesting vector pVUB3:COOHgp63, Cote-Sierra *et al.,* 1998) into the 6xhis-NL-OprI producing pCIMM2 plasmid (Fig. 2) using standard methods en the resulting plasmid was subsequently transformed into chemocompetent *E.coli* cells.

### Construction of 6xhis-tagged antigens.

The recombinant 6xhis-COOHgp63 protein (Indicated as COOHgp63; Fig. 3) was generated by directionally cloning the *BgIII-HindIII* COOHgp63 DNA fragment (generated by digesting vector pVUB3:COOHgp63) into the expression vector pQE32 (Qiagen GmbH, Germany) digested with *BamHI* and *HindIII.*
The recombinant His-tagged FMDV 3D protein was generated by directionally cloning a *BamHI-PstI* FMDV-3D amplicon (generated by PCR amplification from the plasmid p7.5/3D15 (provided by Dr. M. Parkhouse, IAH, Pirbright, U.K.) using 3D-specific primers containing the *BamHI* or *PstI* restriction site coding sequence, respectively) into the expression vector pQE30 (Qiagen GmbH, Germany), restricted with the same enzymes. The resulting ligation mixture was subsequently transformed into chemocompetent *E. coli* cells using standard procedures.

### Expression and purification of recombinant antigens.

Induction of L-Oprl, L-Oprl fusion proteins, 6xhis-NL-Oprl, 6xhis-NL-Oprl fusion proteins and 6xhis-tagged proteins with IPTG and preparation of outer membrane fractions was performed as described previously (Cornelis *et al.,* 1996). Oprl and Oprl fusion proteins are purified from outer membrane fractions solubilized in a buffer containing 50mM Tris-HCI pH 8.0, 0.6% SDS, 10 mM β-mercaptoethanol. The outer membrane proteins were loaded onto a preparative SDS-polyacrylamide column and purified by continuous elution electrophoresis using the Bio-Rad Model 491 Prep Cell (Bio-Rad Laboratories, Hercules, California) according to the manufacturer's instruction. 6xhis-tagged proteins, 6xhis-NL-Oprl and 6xhis-NL-Oprl fusion proteins were purified by affinity chromatography (IMAC) under denaturing conditions using the Ni-NTA superflow resin (Qiagen GmbH, Germany) or TALON Metal Affinity resin (Clontech, Palo Alto, USA) and concentrated by using a VIVASPIN concentrator (VIVASCIENCE, Lincoln, UK), previously treated with 0.02% pluronic acid for 10 min (in the case of L-Oprl and L-Oprl fusion proteins). When necessary, IMAC-purified proteins were re-purified by continuous elution electrophoresis as mentioned above. Finally, proteins were subjected to two successive gel filtration chromatographies in the AKTA explorer (Amersham Pharmacia/Biotech, Sweden) using Superdex-75 HR10/30 (Pharmacia Biotech, Sweden) in order to remove LPS (Hoekstra *et al.,* 1976), and eluted in a buffer containing 20 mM Tris-HCl, pH 8.0, 100mM NaCl, 20 mM glycine and 0.01% SDS. Protein concentration was determined using the Bio-Rad DC Protein Assay (Bio-Rad Laboratories, Hercules, California). Lipopolysaccharide (LPS) in the protein suspension was determined by the Limulus Amebocyte Lysate Assay (Biowhittaker, Inc. Walkersville, MD).

### Transformation of recombinant plasmid into attenuated Salmonella.

Plasmid DNA was transformed into the respective *Salmonella* strains by electroporation using standard procedures (2.5 kV, 400 Ω, 25 µF). After electroporation, the bacterial cells were grown for 2 h at 37°C in LB medium. Aliquots of the transformation mix were grown on LB agar plates containing the appropriate antibiotic to select for recombinant bacteria.

### Production of Salmonella lysates

*Salmonella* cultures were grown overnight in LB medium at 37°C. The bacteria were pelleted, resuspended in PBS supplemented with protease inhibitors (Boehringer Mannheim) and subjected to 3 freeze-thaw cycles followed by sonication. After removing debris by centrifugation, the lysates were aliquoted and stored at -80°C until use.

### Production of Salmonella live oral vaccines

Cultures of recombinant *Salmonellae* were statically grown overnight at 37°C and used to seed fresh medium at a starting dilution of 1:50, and the subcultures were incubated at 37°C until the OD₆₀₀ reached between 0.7-0.75. Expression of the recombinant L-Oprl protein was then achieved by inducing the culture for 30 min with 1 mM IPTG. After induction, the bacteria were pelleted and resuspended to the appropriate cell density in PBS.

### Immunizations with 6xhis-protein, L-Oprl-, and NL-Oprl formulations.

BALB/c, C57BU6 or C57BU6 TNF-α^{-/-} mice were subcutaneously immunized three times at 10 days interval in the base of the tail with 1 µg of either 6xhis-protein, L-Oprl- or NL-Oprl formulation. Preimmune sera were taken one day before the first immunization.

Seven or 10 days after the first or third immunization respectively, mice were killed. Sera, spleens and draining lymph nodes (sacral lymph nodes) were taken to analyze the immune response.

### Cytokine assays.

Homogeneous lymph node and spleen cell suspensions from individual mice were prepared in supplemented RPMI 1640 medium (fetal calf serum 10%, penicillin-streptomycin 100 U and 100µg/ml respectively, L-glutamine 2 mM, 2-mercaptoethanol 5x10⁻⁵ M, MEM amino acid solution 1X and sodium pyruvate 1 mM). 2x10⁶ cells were separately stimulated with or without the appropriate antigen at 37°C in 24-well flat bottom tissue culture plates (Becton Dickinson, Franklin Lakes, NJ, USA). IFN-γ, IL-4, and IL-10 were determined in culture supernatants taken 24, 48, 72 and 96 hours after priming. The cytokine levels were analyzed by a sandwich enzyme-linked immunosorbent assay (ELISA) in accordance to the supplier's instructions (Pharmingen, San Diego, CA USA). Data are represented as mean cytokine concentrations over 4 days.

### Measurement of antibody titers.

Immunoglobulin isotype titers in the preimmune and immune sera were measured by using ELISA (Southern Biotechnology Associates, Inc. Birmingham, AL, USA). Briefly, 96-well Nunc-Immuno plates (Nalge Nunc International, Denmark) were coated with the appropriate antigen and after exposure to diluted preimmune or immune sera, bound antibodies were detected by HRP-labeled goat anti-mouse lgG1, IgG2a, IgG2b, IgG3, IgA and IgM. ELISA titers were specified as the last dilution of the sample whose absorbance was above threefold the preimmune sera value.

### Induction of TNF-α production in peritoneal exudate cells (PECs) after antigen stimulation.

PECs from LPS-resistant C3H/HeJ mice were harvested by washing the peritoneal cavity with 10 ml of ice-cold sucrose solution (0.34 M). The cells were washed in supplemented RPMI 1640 and left to adhere for 2 h. at 37°C in 24-well flat bottom tissue culture plates (Becton Dickinson, Franklin Lakes, NJ, USA) at a concentration of 1x10⁶ cells/ml. The peritoneal exudate cells were stimulated or not with recombinant murine IFN-γ (Life Technologies, Ltd., Paisley, Scotland, 100 U/ml) in the presence of L- and NL-OprlCOOHgp63 or COOHgp63. After overnight incubation in a humidified atmosphere of 5% of CO₂, supernatants were collected to determine TNF-α concentrations by using the DuoSet ELISA development system (R&D systems, Abingdon, UK).

### L. major challenge.

Groups of 15 BALB/c mice were subcutaneously immunized three times with 1 µg of either L-OprlCOOHgp63, NL-OprlCOOHgp63 or COOHgp63. A control group was immunized with the buffer in which the proteins were dissolved. 10 days after the third dose, mice were s.c. challenged with 10⁶ live virulent *L. major* promastigotes in the base of the tail. Progress of the disease was monitored weekly by scoring the lesion development.

### CTL assay

CTLs were derived from spleens of mice that had been immunised with the respective OVA₂₅₇₋₂₆₄ peptide (SIINFEKL)/adjuvant or OVA protein/adjuvant formulations. Starting 10 days after the last immunisation, CTLs were restimulated in vitro by incubating (1-2) 10⁸ spleen cells with 10⁷ irradiated (7000 rad) RMA-S/B7 cells loaded with OVA₂₅₇₋₂₆₄ peptide (SIINFEKL), in 50 ml RPMI complete medium, supplemented with 1 mM non-essential amino acids (Gibco), 1 mM sodium pyruvate (Gibco) and 20 µM 2-mercaptoethanol (Merck) for 5 days at 37°C. After isolation of blasts on Ficoll-Paque (Pharmacia Biotech, Uppsala, Sweden) gradient, the percentage specific lysis was determined in an ¹¹¹In release assay (Kupiec-Weglinski *et al.,* 1988), which is similar to the standard ⁵¹Cr-release assay. Briefly, target cells (RMA-S, RMA-S cells loaded with OVA₂₅₇₋₂₆₄ peptide (SIINFEKL)) labeled with ¹¹¹In were incubated with CTL indifferent ratios for 4 h at 37°C and release of ¹¹¹In in the supernatant was measured. The percentage specific lysis was determined as [(release-spontaneous release)/(maximal release-spontaneous release)]. For maximal release, sodium dodecyl sulphate was added to the target cells (2% final concentration).

### Example 1: The lipid moiety of Oprl-COOHGp63 fusion protein is required for the induction of Type-1 immune responses.

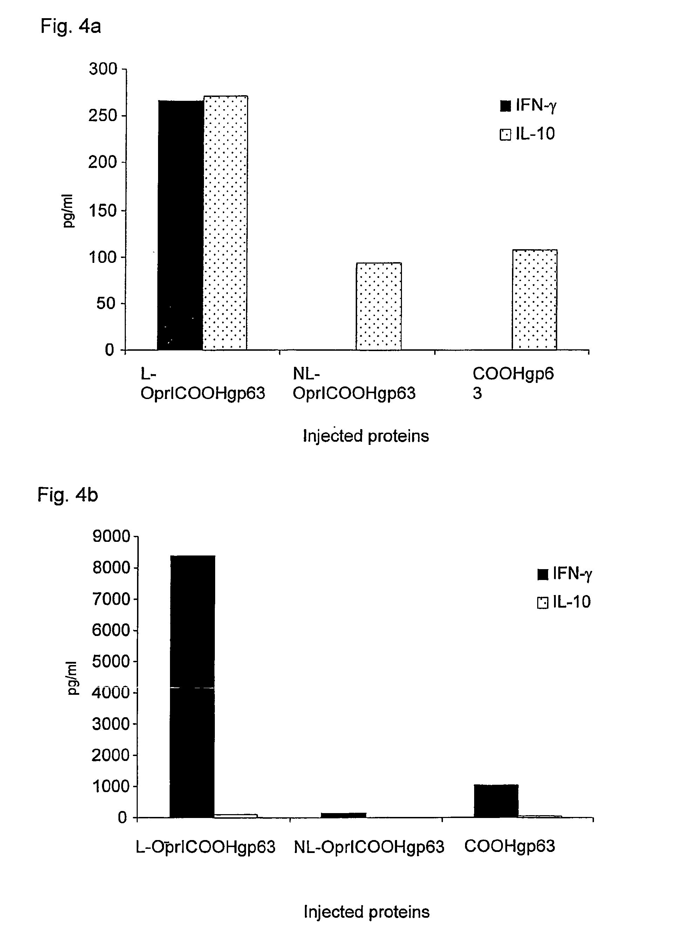

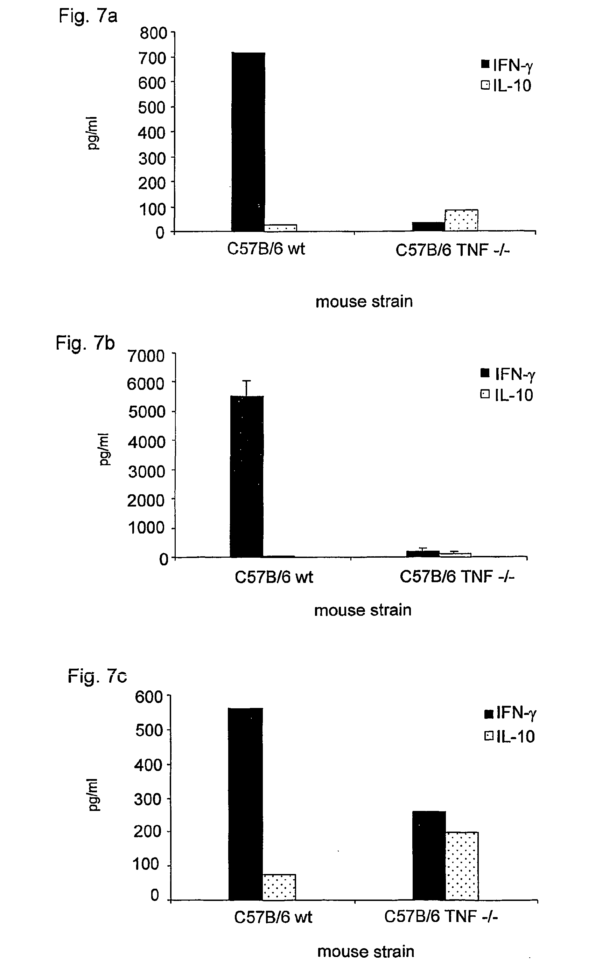

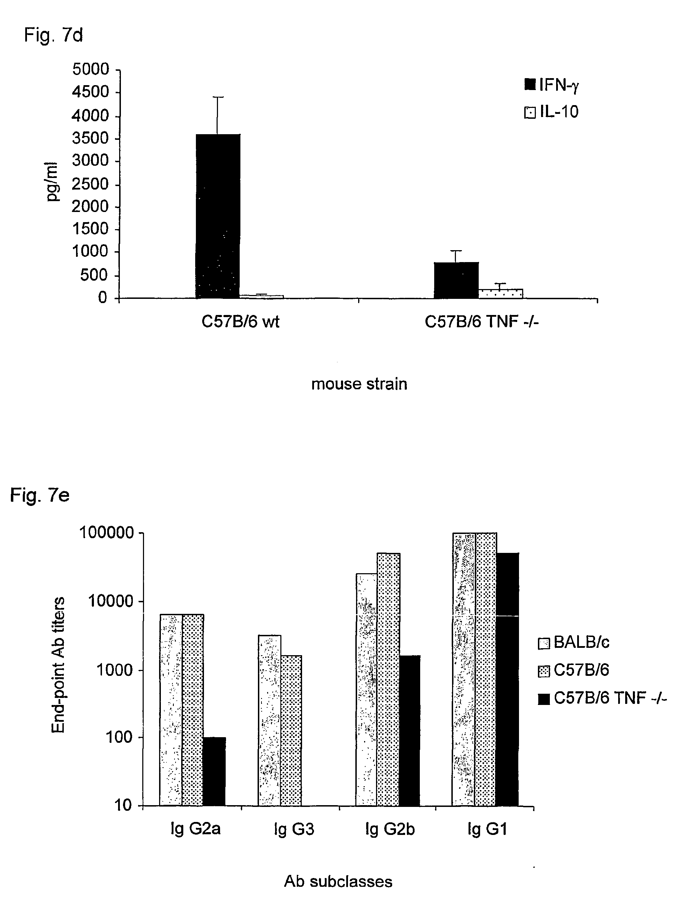

To evaluate the potential adjuvant capacity of the lipoprotein I of *P. aeruginosa* to heterologous proteins and the contribution of its lipid moiety to the immunogenicity of the chimeric Oprl-COOHgp63 lipoprotein, three different recombinant proteins were produced: the lipidated L-OprlCOOHgp63, the non-lipidated NL-OprlCOOHgp63 and the COOHgp63 (Fig 3). All three recombinant proteins contain the COOH-terminal domain of the glycoprotein Gp63 of *L. major,* which contains the host-protective T cell epitopes (Yang *et al.,* 1991). Mice (BALB/c, C57BU6) were immunized subcutaneously once or three times with the recombinant proteins to respectively analyze the early cellular immune responses in the draining lymph nodes, and the secondary humoral immune responses, elicited against the heterologous COOHgp63 antigen. BALB/c is a highly susceptible mouse strain for *L. major* infection and an effective vaccine requires the induction of an IFN-γ- dependent Type-1 immune response (Reiner and Locksley, 1995; Milon *et al.,* 1995). *In vitro* restimulation with the COOHgp63 of lymph node cells from BALB/c mice immunized once with either type of lipoprotein construct or COOHgp63, resulted in a clear induction of IL-10 secretion (Fig 4a). In contrast, only lymph node cells from L-OprlCOOHgp63- immunized BALB/c mice secreted IFN-γ (Fig. 4a). Likewise, in the C57BU6 strain, only lymph node cells from animals immunized with L-OprlCOOHgp63 produced very high levels of IFN-γ upon COOHgp63 restimulation (Fig 4b). The induction of IFN-γ production was sustained after three immunizations as evidenced by the production of high IFN-γ levels in the spleen compartment, whereas the induction of IL-10 production was completely abrogated (Fig. 7.d). When IL-4 was measured in the same culture supernatants, a secretion pattern similar to iL-10 was seen. However the levels of IL-4 production were either undetectable or much lower than the levels of IL-10.

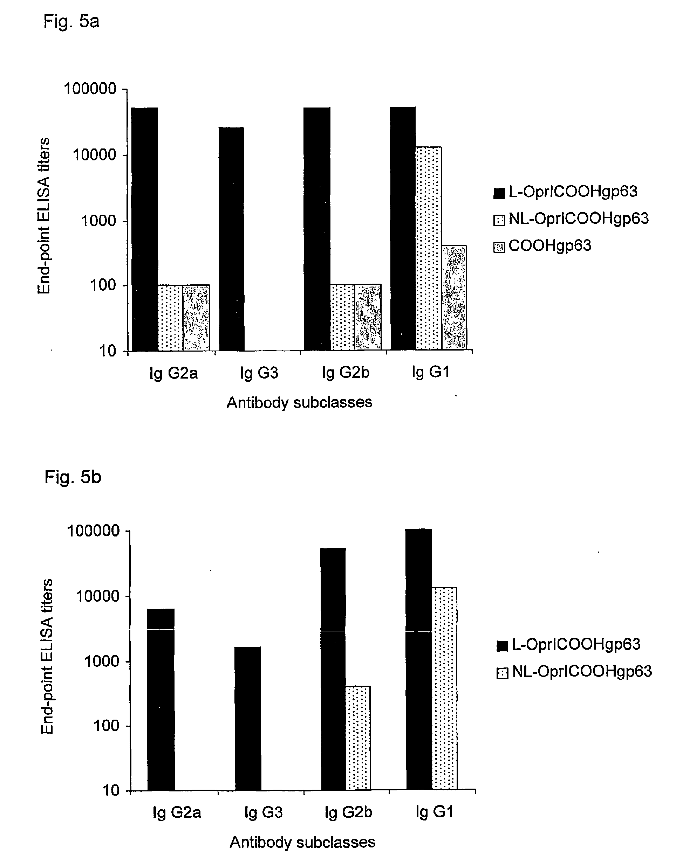

Antibody isotype responses against the COOHgp63 protein were also analyzed in immunized animals. As shown for BALB/c (Fig. 5a) and C57BU6 mice (Fig. 5b), three immunizations with the lipidated Oprl-COOHgp63 induced a significant production of COOHgp63-specific IgG2a, IgG3, IgG2b and IgG1 antibodies. In sharp contrast, the non-lipidated Oprl-COOHgp63 and the COOHgp63 (the latter only shown for BALB/c mice) only induced significant levels of IgG1 anti-Gp63 antibodies and very low or undetectable levels of IgG2a, IgG3 and IgG2b in either mouse strain. There was no detectable IgA in the serum samples while the levels of IgM were marginal. Collectively, these immunization experiments demonstrate that the lipid tail of OprlCOOHgp63 chimeric proteins elicit potent cellular (IFN-γ) and humoral (IgG2a and IgG3 antibodies) Type-1 immune responses.

Comparative analysis of lipidated OprlCOOHgp63, the non-lipidated counterpart and COOHgp63 recombinant proteins in immunised mice demonstrated the crucial importance of the lipid tail of the *P. aeruginosa* lipoprotein I in inducing Type-1 immune responses against the heterologous antigen as evidenced by the cytokine pattern and profile of antibody subclass production. Indeed, a single immunization with the lipidated L-OprlCOOHgp63 biased the T-cell response towards IFN-γ production indicating a preferential induction of a Type-1 immune response. Besides the induction of IFN-γ-producing cells, our results also demonstrate that the lipid tail of Oprl potentiates the induction of humoral responses against a heterologous antigen since immunizations with L-OprlCOOHgp63 increased or triggered IgG2a, IgG3 and IgG2b subclass responses against COOHgp63.

### Example 2: The Type-1 inducing potential of L-OprlCOOHgp63 is TNF-α-dependent.

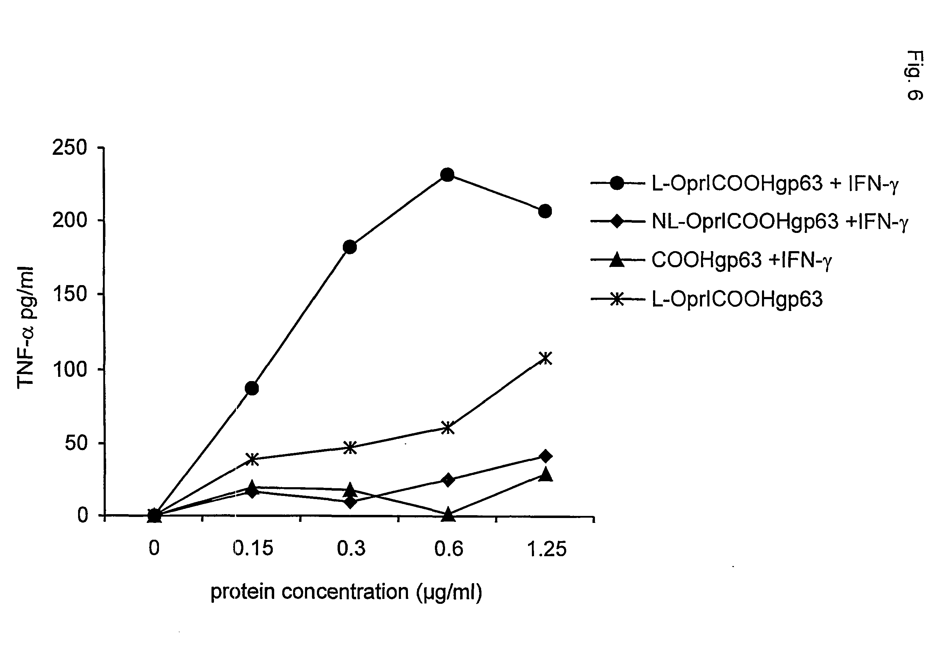

TNF-α, secreted by lipoprotein-activated macrophages (Radolf *et al.,* 1991; Vidal *et al.,* 1998), has been suggested to be a key molecule, together with IL-12, in the induction of IFN-γ production and amplification of Type-1 immune responses (Butler *et al.,* 1999; Tripp *et al.,* 1993). Therefore, it was of interest to test whether (i) Opri-based lipoproteins induce TNF-α production by macrophages and (ii) TNF-α contributes to the Type-1 adjuvant activity of Oprl. Macrophages (the plastic adherent fraction of peritoneal exudate cells (PEC), unactivated or activated with 100 units/ml IFN-γ) from endotoxin-resistant C3H/HeJ mice were stimulated *in vitro* with either the lipidated COOHgp63, non-lipidated COOHgp63 or the COOHgp63 antigen. As shown in Fig. 6, a dose-dependent induction of TNF-α in unprimed macrophages was recorded with the lipidated L-OprlCOOHgp63.

Moreover, the TNF-α-inducing activity of L-OprlCOOHgp63 was strongly increased in IFN-γ-primed macrophages (Fig 6). In these experimental conditions, both the non-lipidated Oprl-COOHgp63 and the COOHgp63 elicited marginal levels of TNF-α synthesis.

To test whether the TNF-α-inducing capacity of L-OprlCOOgp63 contributes to its Type-1 immune response-inducing potential, one and three immunizations with L-OprlCOOHgp63 were performed in C57BU6 TNF-α^{-/-} mice. As shown in Fig. 7, both early and late priming of COOHgp63-specific IFN-γ production was markedly reduced in the culture supernatants of draining lymph node (Fig.7a) or spleen cells (Fig.7b) from L-OprlCOOHgp63-immunized TNF-α^{-/-} mice as compared to immunized C57BI/6 wild-type mice. Likewise, decreased Type-1 responses were also recorded in the culture supernatants of draining lymph node (Fig. 7c) and spleen cells (Fig 7d) from TNF-α^{-/-} mice immunized three times with the antigen, and restimulated *in vitro* with COOHgp63. Analysis of the humoral responses elicited with L-OprlCOOHgp63 (after three immunizations) in BALB/c, C57BU6 wild type and C57BU6 TNF-α^{-/-} mice, revealed that anti-COOHgp63 IgG3 and IgG2a responses were severely reduced in C57BU6 TNF-α^{-/-}mice (Fig 7e). In contrast, the magnitude of IgG1 and IgG2b subclass responses were respectively unaffected or less impaired in immunized C57BU6 TNF-α^{-/-} mice as compared to wild type C57BU6 and BALB/c mice. Altogether, these data suggest that the Type-1 immune response elicited by Oprl is strongly TNF-α-dependent.

The capacity of L-OprICOOHgp63 to instruct acquired immune responses may reflect its potential to trigger innate immune cells. Corroborating other reports that bacterial lipoproteins are potent inducers of TNF-α production (Radolf *et al.,* 1991; Vidal *et al.,* 1998), our results show that only L-OprlCOOHgp63 was capable to stimulate significantly TNF-α production by either naive or IFN-γ-primed macrophages. Local production of TNF-α may in turn signal the development of Type-1 acquired immune responses. Indeed, this cytokine was documented to induce the expression of B7-like costimulatory signals (Swallow *et al.,* 1999), IFN-γ production by T cells (Butler *et al.,* 1999; Darji *et al.,* 1996) and NK cells (Tripp *et al.,* 1993) and Type-1 antibody subclass responses (i.e. IgG2a) (Pasparakis *et al.,* 1996). The involvement of TNF-α in the genesis and/or progression of cellular and humoral Type-1 acquired immune responses to leishmanial antigens is herein further substantiated since both Type-1 cytokine (IFN-γ) and humoral subclass (IgG3 and IgG2a) responses against the heterologous antigen were severely compromised in L-OprlCOOHgp63-immunized TNF-α^{-/-} mice. Hereby, it should be emphasized that CFA-aided immunization did not reveal similar defects in TNF-α^{-/-} mice. Hence, the defective induction of Type-1 responses recorded in L-OprlCOOHgp63 immunized TNF-α^{-/-} mice most probably reflects the TNF-α-inducing potential of Oprl by virtue of its lipid tail. According to our *in vivo* results TNF-α can be considered as a component of the innate immune system which, synergistically with or alternatively to IL-12, bridges the gap between innate and acquired immunity. Finally, since the TNF-α-inducing capacity of Oprl is strongly increased upon macrophage-priming with IFN-γ, TNF-α-mediated induction of IFN-γ production by Oprl-based vaccines may further amplify ongoing or subsequent Oprl elicited immune responses.

### Example 3: Vaccinations with Oprl-based COOHgp63 lipoproteins protect highly susceptible BALB/c mice against Leishmania challenge.

It is well established that during infection with *L. major,* resistant C57BU6 mice mount a polarized Type-1 cellular immune response mediated by IFN-γ production (Reiner and

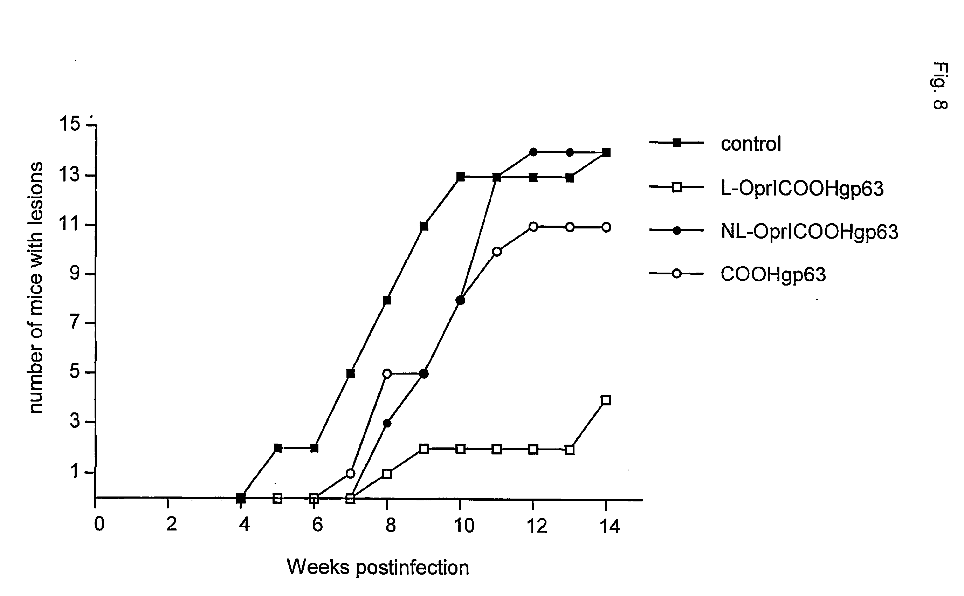

Locksley, 1995; Milon *et al.,* 1995). In view of the capacity of the lipid-modified OprlCOOHgp63 to skew the immune response towards a IFN-γ-producing Type-1 immune response, it was of interest to test whether vaccinations with this lipoprotein could provide protection in highly susceptible BALB/c mice against *Leishmania* challenge. To this end, mice were vaccinated with the lipidated OprlCOOHgp63, the non-lipidated counterpart or COOHgp63 in order to compare the effect of immunization on lesion development. As shown in Fig. 8, a clear delay in the onset of skin lesions in mice vaccinated with the lipid-modified protein was observed. In the groups vaccinated with the non-lipidated OprlCOOHgp63 and the COOHgp63, the pattern of disease appearance was similar to the control group although a slight delay was observed. After 14 weeks of infection, 73% of L-OprlCOOHgp63 vaccinated animals still remained healthy, indicating that vaccination with the lipid-modified protein delayed the appearance of the disease and induced a protective immunity in the majority of the animals.

It is well established that immunological control of *L. major* infections depends on the production of IFN-γ that activates macrophages to kill the parasites via induction of NO production (Milon *et al.,* 1995; Mossalayi *et al.,* 1999; Green *et al.,* 1990). Accordingly, the capacity of L-OprICOOHgp63 to elicit COOHgp63-specific IFN-γ-producing memory cells is reflected by the induction of protective immunity against *L. major* infections in the highly susceptible BALB/c model. Taking into account that this type of immunization is highly TNF-α-dependent, it is worth mentioning that vaccination with leishmanial antigens together with TNF-α prevents disease enhancement and induces protective immunity against L. *major* infection in susceptible BALB/c mice (Liew *et al.,* 1991).

### Example 4: induction of Type-1 immune responses against a heterologous antigen by immunization with a host cell expressing a Oprl-heterologous antigen fusion protein

To see whether L-Oprl, pathogen-derived antigens/peptides, presented in the context of L-Oprl at the surface of live host cells, can induce a relevant immune response, a live vaccination experiment was carried out using a L-Oprl/FMDV antigen presented at the surface of attenuated *Salmonella typhimurium* SL3261 (Hoiseth & Stocker, 1981).

BALB/c mice (8 weeks of age) were immunized internasally with 10⁸ *S. typhimurium* SL3261(pVUB3:3D-15) in a 10µl volume (5 µl per nostril). Three months after the intranasal immunization, mice were killed. Sera and spleens were taken to analyze the immune response.

Homogeneous spleen cell suspensions from individual mice were prepared in supplemented RPMI 1640 medium. 2 x 10⁶ cells were restimulated with either 6 x his-3D, NL-OprI, ovalbumin (irrelevant antigen) or crude *Salmonella* SL3261 lysate. The cytokine levels were determined in the culture supernatants taken 24, 48, 72 and 96 h after restimulation. Antibody isotypes against recombinant 6xhis-3D protein, NL-OprI or crude *Salmonella* SL3261 lysate were determined in naive and immune sera by ELISA. Serum was applied to every separate antigen (2µg/ml) and detected with specific anti-isotype antibodies. Total anti-3D IgG titers are in the range of 1/10000.

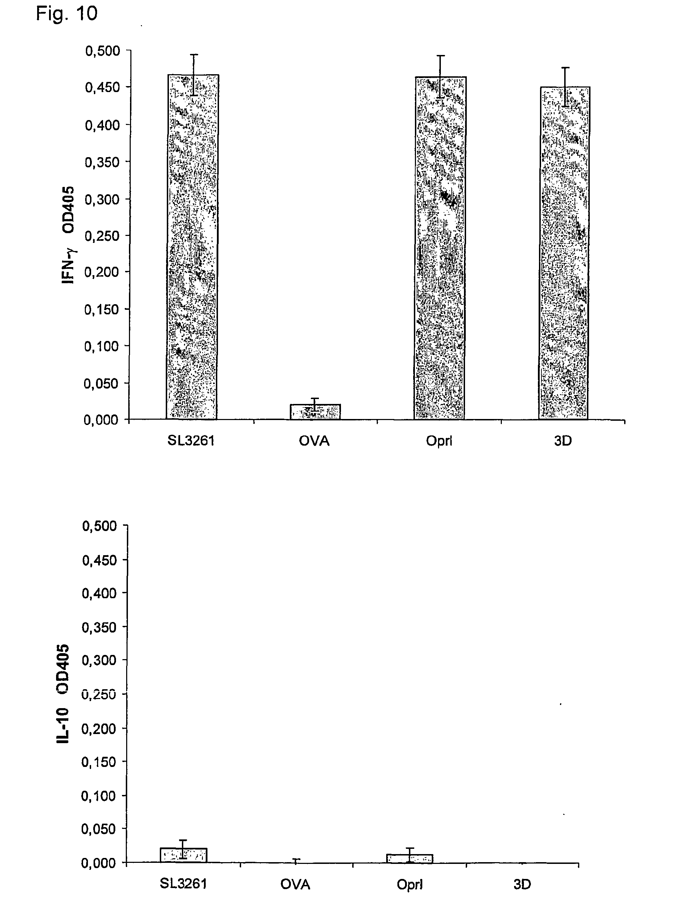

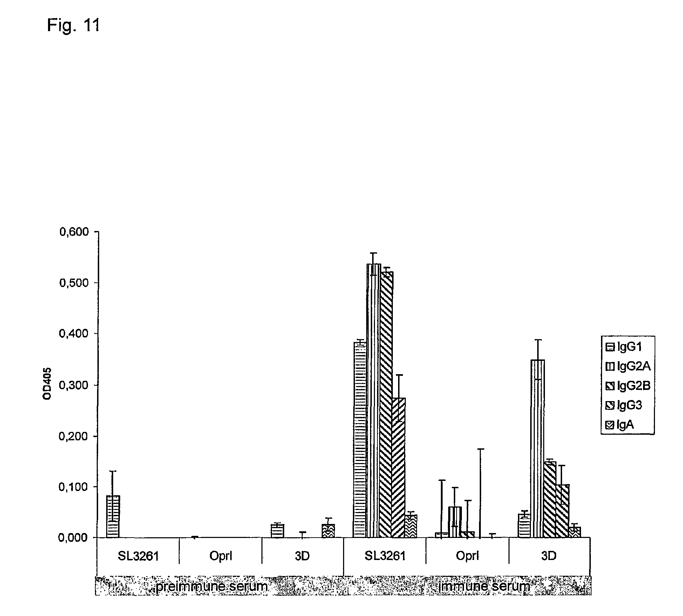

A single immunization with SL3261(pVUB3:3D-15) biased the T-cell response against the heterologous 3D antigen towards IFN-γ production (Fig.10) indicating a preferential induction of a Type-1 immune response. Besides the induction of IFN-γ producing cells, this immunization also elicited a selective humoral (lgG2a) type-1 immune response against 3D (Fig. 11).

### Example 5: Oprl derived fusion proteins induce a long lasting Type-1 immune response

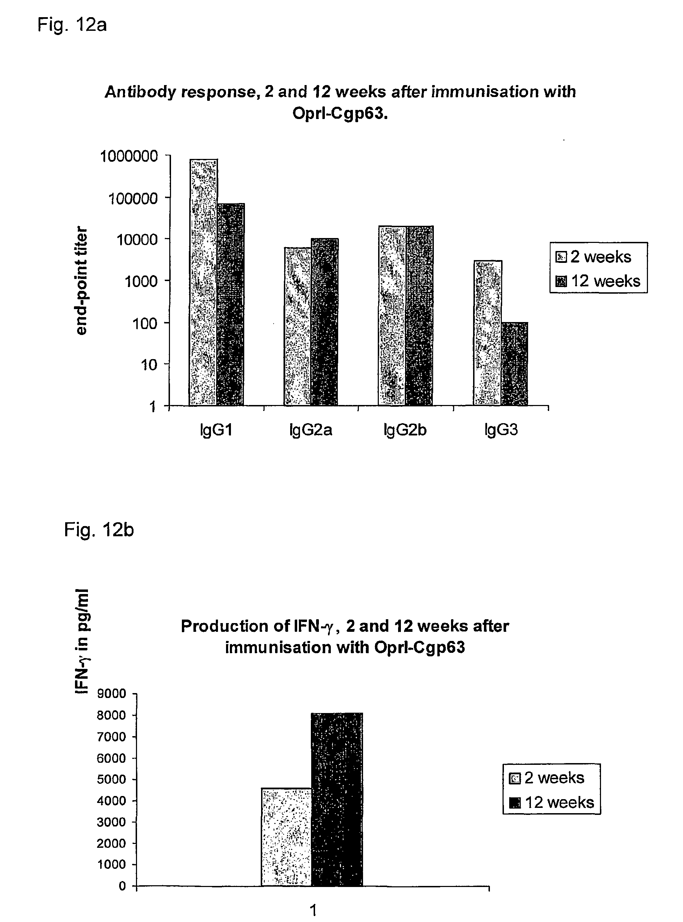

BALB/c mice were s.c. immunised 3 x at 10-days interval with 1 µg Oprl-COOHgp63 antigen. Spleen cells and sera were taken at 2 and 12 weeks after the last immunisation and analysed for the production of cytokines and antibodies resp. The results are shown in Fig. 12, indicating that both antibody response and IFN-γ production is hardly affected by the time.

### Example 6: Oprl retains its Type-1 adjuvanticity when admixed with a heterologous antigen as demonstrated in the Leishmania model

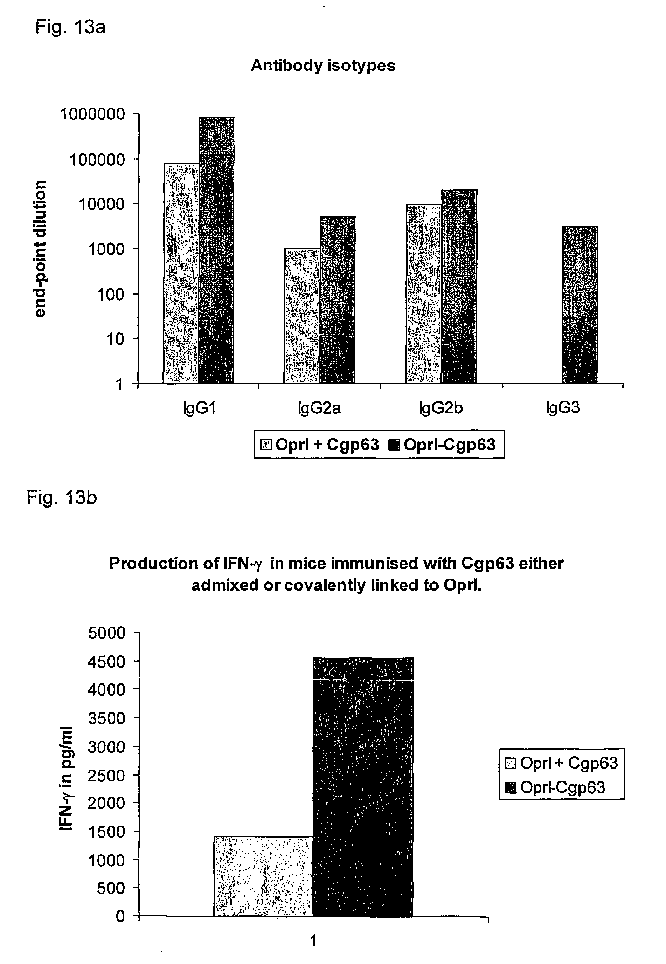

BALB/c mice were s.c. immunised 3 x at 10-days interval with 1 µg OprI-COOHgp63 antigen or L-OprI + 6xhis-COOHgp63 at a same molar basis as the covalent formulation. Spleen cells and sera were taken 2 weeks after the last immunisation and analysed for the production of cytokines and antibodies resp. The results are shown in Fig.13. Although there are some differences in IgG3 response and IFN-γ production, it is clear that the reponse obtained by the Oprl / COOHgp63 mixture is mainly a Type-1 response.

### Example 7: L-Oprl incytes cytolytic CD8 T cells towards MHC-class-I restricted T cell epitopes in a Th cell-independent way.

In view of the capacity of L-Oprl to induce Type-1 humoral and cellular responses against a heterologous antigen fused to its C-terminal or admixed with, it was of interest to look for the possible adjuvant capacity of L-Oprl to induce specific cytotoxic T lymphocytes against a heterologous antigen or peptide. To this end, we evaluated the CTL inducing capacity of L-Oprl in a OVA-model when admixed with free protein or peptides as compared to other adjuvants.

C57BI/6 mice were immunised with i) 1 µg L-Oprl + 1µg OVA protein, ii) 1 µg Oprl + 5 µg OVA ₂₅₇₋₂₆₄ MHC class I (K^{b}-restricted) peptide, iii) 1 µg Oprl + 5 µg OVA₂₅₇₋₂₆₄ MHC class I (K^{b}-restricted) peptide + 5 µg OVA₂₆₅₋₂₈₀ (MHC class II (I-Ab-restricted) Tₕ peptide, iv) PBS + 5 µg OVA₂₅₇₋₂₆₄ MHC class I (K^{b}-restricted) peptide, v) CFA + 5 µg 5 µg OVA₂₅₇₋₂₆₄ MHC class I (K^{b}-restricted) peptide and vi) 1 µg OVA in PBS).

Mice were immunised three times sub-cutaneously (s.c.) at the base of the tail, with 10 days interval. The CTL assay was set up 10 days after the last immunisation. Each mouse was analysed individually, each group consisted of 4 mice.

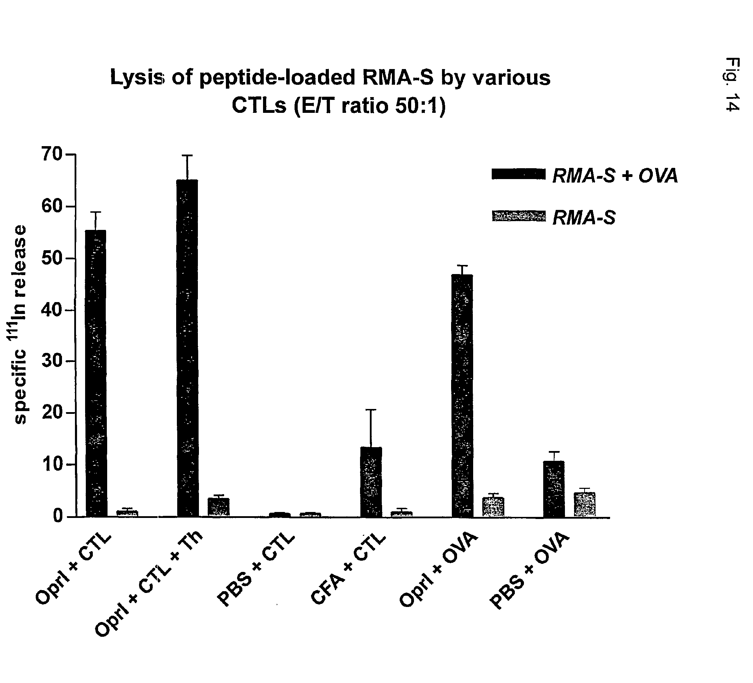

As shown in Fig. 14, splenocytes from all groups of mice immunized with Oprl + antigen/peptide, lysed significantly OVA 257-264 peptide-loaded RMA-S target cells as compared to the unloaded RMA-S target cells. Immunisation with antigen/peptide in PBS did not induce a specific cytolytic activity. As compared to CFA, Oprl seems to be a more potent adjuvant for the induction of CTLs against a minimal CTL epitope.

In conclusion, L-Oprl was shown to incite cytolytic CD8 T cells toward MHC class I-restricted T cell epitopes in a Th cell-independent manner, which could be further potentiated by the addition of T-helper epitope.

### Example 8: Live oral vaccination with recombinant Salmonella expressing an Oprl-rotavirus recombinant antigen, elicits specific neutralizing antibodies against rotavirus

To see whether live oral vaccination using L-Oprl as a carrier for the presentation of heterologous antigens on the surface of a host cell, can indice the appropriate immune response, immunisation experiments were performed with *S. typhimurium* χ4064 (Curtiss & Kelly, 1987) harboring pVUB3:VP8 rotavirus antigen. BALB/c mice were immunised once intranasally with recombinant *S*. *typhimurium* (strain χ4064) expressing i) L-Oprl-rotavirus (VP8) recombinant antigen, ii) L-Oprl or *S. typhimurium* _{χ}4064 alone. Subsequent analysis of the serum taken from all groups of mice revealed the presence of VP8-specific antibodies in the group immunised with *S. typhimurium* _{χ}4064 expressing L-Oprl-rotavirus (VP8) recombinant antigen. To see whether the elicited VP8-specific antibodies could neutralize rotavirus strain RF78 (kindly provided by Dr. Cohen, INRA, France), 100 pfu of rotavirus strain RF78 was mixed with different dilutions of sera collected from mice immunized with either *S.typhimurium* _{χ}4064, _{χ}4064(pVUB3), or _{χ}4064(pVUB3-VP8), and tested for plaque reduction. Polyclonal antibodies against RF78 (A polyclonal serum against rotavirus strain RF78 was prepared and provided by Dr. Cohen, INRA, France) were used as a positive control. As can be seen from Fig. 16, serum from χ4064(pVUB3-VP8) immunised mice could partially neutralise the rotavirus. The titer of neutralization was determined as 60% of plaque reduction.

### References

- Aliprantis, A.O., Yang R.B., Mark, M.R., Suggett, S., Devaux, B., Radolf, J.D., Klimpel, G.R., Godowski, P. & Zychlinsky, A. Cell activation and apoptosis by bacterial lipoproteins through toll-like receptor-2. Science 285, 736-739, (1999).
- BenMohamed, L. Gras-Masse, H., Tartar, A., Daubersies, P., Brahimi, K., Bossus, M., Thomas, A. & Druilhe P. Lipopeptide immunization without adjuvant induces potent and long- lasting B, T helper, and cytotoxic T lymphocyte responses against a malaria liver stage antigen in mice and chimpanzees. Eur.J.Immunol. 27, 1242-1253, (1997).
- Bessler, W.G., Suhr, B., Buhring, H.J., Muller, C.P., Wiesmuller, K.H., Becker, G. & Jung, G. Specific antibodies elicited by antigen covalently linked to a synthetic adjuvant. Immunobiology 170, 239-244, (1985).
- Brightbill, H.D., Libraty, D.H., Krutzik, S.R., Yang, R.B., Belisle, J.T., Bleharski, J.R., Maitland, M., Norgard, M.V., Plevy, S.E., Smale, S.T., Brennan, P.J., Bloom, B.R., Godowski, P.J. & Modlin, R.L.. Host defense mechanisms triggered by microbial lipoproteins through toll-like receptors. Science 285, 732-736, (1999).
- Butler, D.M., Malfait, A.M., Maini, R.N., Brennan, F.M. & Feldmann, M. Anti-IL-12 and anti-TNF antibodies synergistically suppress the progression of murine collagen-induced arthritis. Eur.J.ImmunoL 29, 2205-2212, (1999).
- Cornelis, P. Sierra, J.C., Lim, A., Malur, A., Tungpradabkul, S., Tazka, H., Leitao, A., Martins, C.V., di Perna, C., Brys, L., De Baetseller, P. & Hamers, R. Development of new cloning vectors for the production of immunogenic outer membrane fusion proteins in Escherichia coli. Biotechnology (N.Y.) 14, 203-208, (1996).
- Cote-Sierra, J., Jongert, E., Bredan, A., Gautam, D.C., Parkhouse, M., Cornelis, P., De Baetselier, P. & Revets, H. A new membrane-bound Oprl lipoprotein expression vector. High production of heterologous fusion proteins in gram (-) bacteria and the implications for oral vaccination. Gene 221, 25-34, (1998).
- Curtiss, R. & Kelly, S.M. Salmonella typhimurium deletion mutants lacking adenylate cyclase and cyclic AMP receptor protein are avirulent and immunogenic. Infect. Immun. 55, 3035-3043, (1987).
- Darji, A., Beschin, A., Sileghem, M., Heremans, H., Brys, L. & De Baetselier, P. In vitro simulation of immunosuppression caused by Trypanosoma brucei: active involvement of gamma interferon and tumor necrosis factor in the pathway of suppression. Infect. Immun. 64, 1937-1943, (1996).
- Deres, K., Schild, H., Wiesmuller, K.H., Jung, G. & Rammensee, H.G. In vivo priming of virus-specific cytotoxic T lymphocytes with synthetic lipopeptide vaccine. Nature 342, 561-564, (1989).
- de Jong, R., Janson, A.A., Faber, W.R., Naafs, B. & Ottenhoff, T.H. IL-2 and IL-12 act in synergy to overcome antigen-specific T-cell unresponsiveness in mycobacterial disease. J. Immunol. 159, 786-793, (1997).
- Donckier, V., Abramowicz, D., Bruyns, C., Florquin, S., Vanderhaeghen, M.L., Amraoui, Z., Dubois, C., Vandenabeele, P. and Goldman, M. IFN-gamma prevents Th2 cell-mediated pathology after neonatal injection of semiallogenic spleen cells in mice. J. Immunol. 153, 2361-2368, (1994).
- Erdile, L.F., Brandt, M.A., Warakomski, D.J., Westrack, G.J., Sadziene, A., Barbour, A.G. & Mays, J.P. Role of attached lipid in immunogenicity of Borrelia burgdorferi OspA. Infect.Immun. 61, 81-90, (1993).
- Fearon, D.T. & Locksley, R.M. The instructive role of innate immunity in the acquired immune response. Science 272, 50-53, (1996).
- Green, S.J., Crawford, R.M., Hockmeyer, J.T., Meltzer, M.S. & Nacy, C.A. Leishmania major amastigotes initiate the L-arginine-dependent killing mechanism in IFN-γ-stimulated macrophages by induction of tumor necrosis factor-alpha. J.Immunol. 145, 4290-4297, (1990).
- Heinzel, F.P., Sadick, M.D., Mutha, S.S. & Locksley, R.M. Production of IFN-γ, IL-2, IL-4 and IL-10 by CD4+ lymphocytes in vivo during healing and progressive murine leishmaniasis. Proc. Natl. Acad. Sci. USA 88, 7011-7015, (1991).
- Hoekstra, D., van der Laan, J.W., de Leij, L. & Witholt, B. Release of outer membrane fragments from normally growing Escherichia coli. Biochim.Biophys.Acta 455, 889-899, (1976).
- Hoiseth, S.K. & Stocker B.A.D. Aromatic-dependent Salmonella typhimurium are non-virulent and effective as live vaccines. Nature 291, 238-240, (1981).
- Infante-Duarte, C. & Kamradt, T. Lipopeptides of Borrelia burgdorferi outer surface proteins induce Th1 phenotype development in αβ T-cell receptor transgenic mice. Infect.Immun. 65, 4094-4099, (1997).
- Ino, M., Nagase, S., Nagasawa, T. Koyama, A. & Tachibana, S. The outer membrane protein I of Pseudomonas aeruginosa PA01, a possible polluant of dialysate in hemodialysis, induces cytokines in mouse bone marrow cells. Nephron, 82, 324-330, (1999).
- Kupiec-Weglinski, J.W., Austyn, J.M. & Morris, P.J. Migration patterns of dendritic cells in the mouse. Traffic from blood, and T cell-dependent and -independent entry to lymphoid tissues. J. Exp. Med. 167, 632-645, (1988).
- Leitao, A., Malur, A., Cornelis, P. & Martins, C.L. Identification of a 25-amino acid sequence from the major African swine fever virus structural protein VP72 recognised by porcine cytotoxic T lymphocytes using a lipoprotein based expression system. J. Virol. Methods 75, 113-119, (1998).
- Leonard, J.P., Waldburger, K.E. & Goldman, S.J. Prevention of experimental autoimmune encephalomyelitis by antibodies against interleukin 12. J. Exp. Med. 181, 381-386, (1995).
- Lex, A., Wiesmuller, K.H., Jung, G. & Bessler, W.G. A synthetic analogue of Escherichia coli lipoprotein, tripalmitoyl pentapeptide, constitutes a potent immune adjuvant. J.Immunol. 137, 2676-2681, (1986).
- Liew, F.W., Li, Y., Yang, D.M., Severn, A. & Cox, F.E. TNF-alpha reverses the disease-exacerbating effect of subcutaneous immunization against murine cutaneous leismaniasis. Immunology 74, 304-309, (1991).
- Milon, G., Del Giudice, G. & Louis, J.A. Immunobiology of Experimental Cutaneous Leishmaniasis. Parasitol.Today 11, 244-247, (1995).
- Mosmann, T.R. & Coffman, R.C. Th1 and Th2 cells: different patterns of lymphokine secretion lead to different functional properties. Annu. Rev. Immunol., 7, 145- (1989).
- Mossalayi, M.D., Arock, M., Mazier, D., Vincendeau, P. & Vouldoukis, I. The human immune response during cutaneous leishmaniasis: NO problem. Parasitol. Today 15, 342-345, (1999).
- Murphy, J.W., Wu-Hsieh, B.A., Singer-Vermes, L.M. Ferrante, A., Moser, S., Ruso, M., Vaz, S.A., Burger, E., Calich, V.L. & Kowanko, I.C. Cytokines in the host response to mycotic agents. J. Med. Vet. Mycol. 32 suppl 1, 203, (1994).
- Nabors, G.S., Afonso, L.C.C., Farrell, J.PO. & Scott, P. Switch from a type 2 to a type 1 T helper cell response and cure of established Leishmania major infection in mice is induced by combination therapy with interleukin 12 and pentosam. Proc. Natl. Acad. Sci. USA, 92, 3142-3146 , (1995).
- Pasparakis, M., Alexopoulou, L., Episkopou, V. & Kollias, G. Immune and inflammatory responses in TNF alpha-deficient mice: a critical requirement for TNF alpha in the formation of primary B cell follicles, follicular dendritic cell networks and germinal centers, and in the maturation of the humoral immune response [see comments]. J.Exp.Med. 184, 1397-1411, (1996).
- Racke, M.K., Bonomo, A., Scott, D.E., Canella, B., Levine, A., Raine, C.S., Shevach, E.M. & Rocken, M. Cytikine induced immune deviation as a therapy for inflammatory autoimmune disease. J. Exp. Med., 180, 1961-1966, (1994).
- Racke, M.K., Burnett, D., Pak, S.H., Albert, P.S., Cannella, B., Raine, C.S., McFarlin, D.E. & Scott, D.E. Retinoid treatment of experimental allergic encephalomyelitis. IL-4 production correlates with improved disease course. J. ImmunoL, 154, 450-458, (1995).
- Radolf, J.D., Norgard, M.V., Brandt, M.E., Isaacs, R.D., Thompson, P.A. & Beutier, B. Lipoproteins of Borrelia burgdorferi and Treponema pallidum activate cachectin/tumor necrosis factor synthesis. Analysis using a CAT reporter construct. J.Immunol. 147, 1968-1974, (1991).
- Reiner, S.L. & Locksley, R.M. The regulation of immunity to Leishmania major. Annu.Rev.Immunol. 13, 151-177, (1995).
- Swallow, M.M., Wallin, J.J. & Sha, W.C. B7h, a novel costimulatory homolog of B7.1 and B7.2, is induced by TNFalpha. Immunity. 11, 423-432, (1999).
- Taniguchi, T., Takata, M., Ikeda, A., Momotani, E. & Sekikawa, K. Failure of germinal center formation and impairment of response to endotoxin in tumor necrosis factor alpha-deficient mice. Lab,Invest, 77, 647-658, (1997).
- Tripp, C.S., Wolf, S.F. & Unanue, E.R. Interleukin 12 and tumor necrosis factor α are costimulators of interferon γ production by natural killer cells in severe combined immunodeficiency mice with listeriosis, and interleukin 10 is a physiologic antagonist. Proc. Nafl. Acad. Sci. USA. 90, 3725-3729, (1993).
- Vidal, V., Scragg, I.G., Cutler, S.J., Rockett, K.A., Fekade, D., Warrell, D.A., Wright, D.J. & Kwiatkowski, D. Variable major lipoprotein is a principal TNF-inducing factor of louse- borne relapsing fever. Nat.Med. 4, 1416-1420, (1998).
- Vogel, G. New clues to asthma therapies. Science, 276, 1643-1646, (1997).
- Weis, J.J., Ma, Y. & Erdile, L.F. Biological activities of native and recombinant Borrelia burgdorferi outer surface protein A: dependence on lipid modification. Infect.Immun. 62, 4632-4636, (1994).
- Yamamura, M., Uyemura, K. and Deans, R.J. Defining protective responses to pathogens: cytokine profiles in leprosy lesions. Science, 254, 277-279, (1991).
- Yang, D.M., Rogers, M.V. & Liew, F.W. Identification and characterization of host-protective T-cell epitopes of a major surface glycoprotein (Gp63) from Leishmania major. Immunology. 72, 3-9, (1991).

## Claims

1. Use of Oprl, or a functional fragment thereof, together with an antigen or allergen for the manufacture of a medicament to prevent or treat a disease caused by abnormal activation of a Th2 type immune response and/or in which the natural Th1 response is insufficient, wherein said disease is TBC, leprosy, a mycotic infection, allergic asthma, an autoimmune disease such as chronic autoimmune graft-versus-host disease or an infection with a rotavirus.

2. Use according to claim 1 wherein Oprl, or a functional fragment thereof, acts as an adjuvant to obtain a Th1 type immune response against a heterologous antigen.

3. Use according to claim 2 wherein said Oprl, or a functional fragment thereof, is fused to said heterologous antigen.

4. Use according to claim 3 wherein an Oprl-heterologous antigen fusion protein is expressed on a host cell.

5. Use of a host cell according to claim 4 for the manufacture of a medicament to prevent or treat a disease caused by abnormal activation of a Th2 type immune response and/or in which the natural Th1 response is insufficient, wherein said disease is TBC, leprosy, a mycotic infection, allergic asthma, an autoimmune disease such as chronic graft-versus-host disease or an infection with a rotavirus.

## Patentansprüche

1. Verwendung von Oprl oder eines funktionellen Fragments davon, zusammen mit einem Antigen oder Allergen, zur Herstellung eines Arzneimittels zum Verhindern oder Behandeln einer Erkrankung, die von abnormer Aktivierung einer Th2-Typ-Immunantwort verursacht wird und/oder in der die natürliche Th1-Antwort unzureichend ist, wobei es sich bei der Erkrankung um TBC, Lepra, eine Mykose, allergisches Asthma, eine Autoimmunerkrankung wie chronische autoimmune Graft-versus-Host-Reaktion oder eine Infektion mit einem Rotavirus handelt.

2. Verwendung nach Anspruch 1, wobei Oprl oder ein funktionelles Fragment davon als ein Adjuvans fungiert, um eine Th1-Typ-Immunantwort gegen ein heterologes Antigen zu erhalten.

3. Verwendung nach Anspruch 2, wobei das Oprl oder ein funktionelles Fragment davon mit dem heterologen Antigen fusioniert wird.

4. Verwendung nach Anspruch 3, wobei ein Fusionsprotein aus Oprl und heterologem Antigen auf einer Wirtszelle exprimiert wird.

5. Verwendung einer Wirtszelle nach Anspruch 4 zur Herstellung eines Arzneimittels zum Verhindern oder Behandeln einer Erkrankung, die von abnormer Aktivierung einer Th2-Typ-Immunantwort verursacht wird und/oder in der die natürliche Th1-Antwort unzureichend ist, wobei es sich bei der Erkrankung um TBC, Lepra, eine Mykose, allergisches Asthma, eine Autoimmunerkrankung wie chronische autoimmune Graft-versus-Host-Reaktion oder eine Infektion mit einem Rotavirus handelt.

## Revendications

1. Utilisation de Oprl, ou d'un fragment fonctionnel de celle-ci, avec un antigène ou allergène pour la fabrication d'un médicament pour empêcher ou traiter une maladie provoquée par l'activation anormale d'une réponse immunitaire de type Th2 et/ou dans laquelle la réponse Th1 naturelle est insuffisante, dans laquelle ladite maladie est TBC, la lèpre, une mycose, l'asthme allergique, une maladie auto-immune telle que la réaction auto-immune chronique de greffe contre hôte ou une infection avec un rotavirus.

2. Utilisation selon la revendication 1, dans laquelle Oprl, ou un fragment fonctionnel de celle-ci, agit comme adjuvant pour obtenir une réponse immunitaire de type Th1 contre un antigène hétérologue.

3. Utilisation selon la revendication 2, dans laquelle ladite Oprl, ou un fragment fonctionnel de celle-ci, est condensée audit antigène hétérologue.

4. Utilisation selon la revendication 3, dans laquelle une protéine de fusion Oprl-antigène hétérologue est exprimée sur une cellule hôte.

5. Utilisation d'une cellule hôte selon la revendication 4 pour la fabrication d'un médicament pour empêcher ou traiter une maladie provoquée par l'activation anormale d'une réponse immunitaire de type Th2 et/ou dans laquelle la réponse Th1 naturelle est insuffisante, dans laquelle ladite maladie est TBC, la lèpre, une mycose, l'asthme allergique, une maladie auto-immune telle que la réaction chronique de greffe contre hôte ou une infection avec un rotavirus.
